# EUROPEAN PATENT APPLICATION

(11) **EP 4 538 388 A1**
(43) Date of publication of application: **16.04.2025**
(21) Application number: 23203060.1
(22) Date of filing: 11.10.2023
(51) Int. Cl.: C12Q 1/6806, C12Q 1/6883, C12Q 1/6886

(54) **QUANTITATIVE ANALYSIS OF SHORT DOUBLE-STRANDED CELL FREE DNA IN LIQUID BIOPSY**

(71) Applicant: Fraunhofer-Gesellschaft zur Förderung der angewandten Forschung e.V., 80686 München (DE)
(72) Inventor: Hartwig, Christina, 70569 Stuttgart (DE); Müller, Jan, 70569 Stuttgart (DE); Sohn, Kai, 70569 Stuttgart (DE); Sonntag, Mirko, 70569 Stuttgart (DE)
(74) Representative: Altmann Stößel Dick Patentanwälte PartG mbB

(57) **Abstract**

The present invention relates to a method for identifying a cfDNA derived biomarker for a physiological condition, disease or disorder of interest, comprising: a) isolating cfDNA from a sample containing cfDNA of a subject known to be in the physiological condition of interest or suffer from the disease or disorder of interest; b) selecting short double-stranded cfDNA fragments; c) determining the nucleic acid sequences of said short double-stranded cfDNA fragments; d) comparing the nucleic acid sequences of said short double-stranded cfDNA fragments to a reference; and; e) based on said comparison in step d) identifying at least one cfDNA derived biomarker associated with the physiological condition, disease or disorder of interest. Moreover, the present invention relates to a method for assessing a physiological condition, disease or disorder of interest in a sample of a subject suspected to suffer therefrom, comprising a) isolating cfDNA from a sample containing cfDNA of said subject; b) selecting short double-stranded cfDNA fragments; c) determining the nucleic acid sequences of said short double-stranded cfDNA fragments; and d) determining the presence, absence or abundance of at least one disease or disorder associated cfDNA derived biomarker associated with the physiological condition, disease or disorder of interest within the determined of the nucleic acid sequences of said short double-stranded cfDNA fragments. Further, the present invention contemplates a method for determining efficacy of treating a physiological condition, disease or disorder of interest in a sample of a subject known to be in said condition or suffer from said disease or disorder comprising: a) isolating cfDNA from a sample containing cfDNA of said subject; b) selecting short double-stranded cfDNA fragments; c) determining the nucleic acid sequences of said short double-stranded cfDNA fragments; d) determining the presence, absence or abundance of at least one cfDNA derived biomarker associated with the physiological condition disease, or disorder of interest within the determined nucleic acid sequences of said short double-stranded cfDNA fragments; e) repeating steps a) to d); and f) determining efficacy of therapy based on the determination over time. Also, the present invention relates to the use of a cfDNA derived biomarker in a sample for assessing a disease or disorder in a subject suspected to suffer therefrom as well as a device for detecting at least one cfDNA derived biomarker in a liquid biopsy sample.

## Description

The present invention relates to a method for identifying a cfDNA derived biomarker for a physiological condition, disease or disorder of interest, comprising: a) isolating cfDNA from a sample containing cfDNA of a subject known to be in the physiological condition of interest or suffer from the disease or disorder of interest; b) selecting short double-stranded cfDNA fragments; c) determining the nucleic acid sequences of said short double-stranded cfDNA fragments; d) comparing the nucleic acid sequences of said short double-stranded cfDNA fragments to a reference; and; e) based on said comparison in step d) identifying at least one cfDNA derived biomarker associated with the physiological condition, disease or disorder of interest. Moreover, the present invention relates to a method for assessing a physiological condition, disease or disorder of interest in a sample of a subject suspected to suffer therefrom, comprising a) isolating cfDNA from a sample containing cfDNA of said subject; b) selecting short double-stranded cfDNA fragments; c) determining the nucleic acid sequences of said short double-stranded cfDNA fragments; and d) determining the presence, absence or abundance of at least one disease or disorder associated cfDNA derived biomarker associated with the physiological condition, disease or disorder of interest within the determined of the nucleic acid sequences of said short double-stranded cfDNA fragments. Further, the present invention contemplates a method for determining efficacy of treating a physiological condition, disease or disorder of interest in a sample of a subject known to be in said condition or suffer from said disease or disorder comprising: a) isolating cfDNA from a sample containing cfDNA of said subject; b) selecting short double-stranded cfDNA fragments; c) determining the nucleic acid sequences of said short double-stranded cfDNA fragments; d) determining the presence, absence or abundance of at least one cfDNA derived biomarker associated with the physiological condition disease, or disorder of interest within the determined nucleic acid sequences of said short double-stranded cfDNA fragments; e) repeating steps a) to d); and f) determining efficacy of therapy based on the determination over time. Also, the present invention relates to the use of a cfDNA derived biomarker in a sample for assessing a disease or disorder in a subject suspected to suffer therefrom as well as a device for detecting at least one cfDNA derived biomarker in a liquid biopsy sample.

As a minimally invasive, diagnostic procedure liquid biopsies are based on various types of biomarkers, including circulating extracellular nucleic acids like cell-free DNA (cfDNA), extracellular vesicles, or circulating tumor cells. Cell-free DNA can be released into the bloodstream by apoptosis, necrosis, or active secretion from almost all cell types and tissues, however, the largest proportion is derived from the hematopoietic system (Sun, et al. 2015). In addition to physiological release from normal cellular turnover, cancer cells or microbial pathogens are also known to release their DNA into bloodstream circulation (Heitzer et al. 2020). Released genomic DNA is then degraded by DNA-digesting enzymes (nucleases), producing fragments mainly 147 to 167 base pairs (bp) in size, corresponding to one nucleosome (Lo et al. 2021). By high throughput sequencing of cfDNA fragments, nucleosome positioning can be inferred at base pair resolution (Snyder et al. 2016). The exact positions of nucleosomes and chromatin structure play a key role in regulating gene expression by providing access to DNA for the transcription machinery. Dense packaging of DNA restricts access by the molecular transcription machinery, while an open structure depleted of nucleosomes makes DNA more accessible for key regulators of transcription, like transcription factors, enhancers, or repressors. Unfortunately, transcription factor footprinting, i.e., the measurement of genome-wide binding of regulatory DNA-binding proteins, cannot be directly analyzed by liquid biopsy approaches, yet. Up to date, the diagnosis of various tumors and inflammatory diseases requires tissue biopsies. Due to their invasive nature, these are associated with risks for patients and do not allow repeated and/or regular and thus dynamic analysis of the clinical indication or of a physiological condition.

Recently, using ultra-deep sequencing of total cell-free DNA, Snyder et al. and Burnham et al. found a minor fraction of double-stranded cfDNA that is significantly shorter than normal cfDNA, ranging from 35 to 80 nucleotides. They proposed that this short cfDNA could be protected by DNA-binding factors and therefore might represent direct transcription factor binding events. However, ultra-deep sequencing of total cfDNA shows limitations as it may not allow for efficient and comprehensive analyses of genome-wide DNA-binding events in liquid biopsies that could be also feasible for routine diagnostic purposes at reasonable costs.

There is thus a need in the art to provide new reliable means and methods for minimally invasive diagnosis. In particular, there is a need to provide means and methods avoiding and overcoming, at least in part, the drawbacks of the prior art as discussed above.

This need is met by methods, uses and device with the features of the independent claims. Preferred embodiments, which might be realized in an isolated fashion or in any arbitrary combination are listed in the dependent claims.

The present inventors have developed an efficient enrichment and analysis platform for short double-stranded cfDNA fragments from liquid biopsy samples such as blood plasma, in particular for those showing a length distribution of 20 to 60 bps. The inventors demonstrate that such so called DNA footprint fragments accumulate at open chromatin as well as gene regulatory elements. Based on this DNA footprint the inventors identified differentially enriched genomic regions and occupied transcription factor motifs between colorectal and pancreatic cancers as well as between septic patients and clinical controls to facilitate diagnostic discrimination of various diseases. The method may also be applied for identifying physiological conditions of a subject. A new biomarker class, the quantitative analysis of short double-stranded cell-free DNA (footprint DNA) by liquid biopsy (liquid footprint), is hereby established.

In accordance, the present invention relates to a method for identifying a cfDNA-derived biomarker for a physiological condition, disease or disorder of interest, comprising:
a) isolating cfDNA from a sample containing cfDNA of a subject known to be in the physiological condition of interest or to suffer from the disease or disorder of interest;
b) selecting short double-stranded cfDNA fragments;
c) determining the nucleic acid sequences of said short double-stranded cfDNA fragments;
d) comparing the nucleic acid sequences of said short double-stranded cfDNA fragments to a reference; and;
e) based on said comparison in step d) identifying at least one cfDNA derived biomarker associated with the physiological condition, disease or disorder of interest.

Said method is advantageous as it provides minimally invasive but reliable and efficient means for adequate diagnosis and differentiation of various diseases and physiological conditions. The interaction of DNA-binding proteins (e.g. transcription factors) and their essential role in genetic regulation may be analyzed *in vivo.*

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a plurality of cells. The term "plurality" is used herein in its common meaning to relate to a quantity of more than one, i.e. at least two. Thus, a plurality may preferably be a number of two or more, three or more, five or more, ten or more, 25 or more, 50 or more 100 or more, 1000 or more. The terms "plurality" and "multitude" are used interchangeably herein unless indicated otherwise.

Further, as used in the following, the terms "preferably", "more preferably", "most preferably", "particularly", "more particularly", "specifically", "more specifically", "typically", "more typically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are *in vitro* methods. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned herein.

As used herein, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, buffer, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The term "cfDNA", as used herein refers to cell-free DNA, also known as circulating free DNA. The term generally encompasses degraded DNA fragments, typically nuclear or mitochondrial DNA fragments, that are released into body fluids. cfDNA can be double-stranded (ds) or single-stranded (ss). In line with the present invention, typically ds cfDNA is selected. Cell-free DNA can be released into the bloodstream or other body fluids by apoptosis, necrosis, or active secretion from almost all cell types and tissues, the largest proportion typically being derived from the hematopoietic system. Commonly, cfDNA can be grouped into regular cfDNA fragments of a typical size between about 100 and 500 bp or even longer, and short cfDNA fragments of a fragment size below 100 bp. Of particular interest according to the present invention are short cfDNA fragments, typically the short double-stranded cfDNA fragments are double-stranded DNA fragments of a fragment size below 100 bp, more typically of a size between 10 and 80 bp, even more typically between 20 and 60 bp.

Short ds cfDNA in line with the present invention may also be referred to as "footprint DNA". "Footprint DNA" is thought by the present inventors, without being bound by theory, to reflect stretches of DNA sites protected by any kind of protein-DNA interaction, for example transcription factor (TF) binding sites, binding sites of structural proteins, binding site of regulatory proteins, transcriptional starts sites, or gene promoter sequences, CpG islands, preferably TF binding sites. Short ds cfDNA is typically characterized by an elevated average GC content. More typically, the GC content is elevated by at least 5 %, at least 7 % at least 10 %, at least 12 % at least 15 % at least 17 % compared to the average GC content of genomic DNA of a reference genome. The GC content of an appropriate reference genome is known to the person of skill in the art. Moreover, the person of skill in the art knows how to select the appropriate reference genome. Typically, the appropriate reference genome is that of the same species as the subject or a species closely related thereto. Specifically, in line with the present invention, the sample is a sample of a human body fluid and the reference genome is the human genome.

The term "fragment" of a biological macromolecule, preferably of a DNA, is used herein in a wide sense relating to any sub-part of the respective biological macromolecule comprising the indicated sequence, structure and/or function. Thus, the term includes sub-parts generated by actual fragmentation of a biological macromolecule, in particular of a DNA molecule, more particularly of a genome, but also sub-parts derived from the respective biological macromolecule in an abstract manner, e.g. *in silico.* In accordance, the term "subsequence" of a biological sequence, preferably of a nucleic acid sequence, relates to any sub-part of the respective biological sequence comprising the indicated sequence and preferably having the indicated function, if indicated. A fragment according to the present invention, in particular relates to a cfDNA fragment as defined elsewhere herein.

The term "nucleic acid", as used herein, refers to a linear or circular nucleic acid molecule in particular to a linear nucleic acid molecule. The term encompasses single- as well as, partially or completely, double-stranded nucleic acid molecules. Preferably, the nucleic acid molecule is a DNA molecule, which may also be referred to as "DNA", more preferably a dsDNA molecule. Moreover, comprised are also chemically modified nucleic acid molecules including naturally occurring modified nucleic acid molecules such as glycosylated or methylated nucleic acid molecules or artificially modified derivatives such as biotinylated p nucleic acid molecules, locked nucleic acids, peptide nucleic acids, and the like. Typically, the DNA molecules according to the present invention are naturally occurring nucleic acid molecule, more typically cfDNA fragments as specified elsewhere herein. Nucleic acid molecules that comprise a multitude of nucleic acids, such as at least ten nucleic acids, are also referred to as polynucleotides.

Unless specifically indicated otherwise, reference to specific polynucleotides herein preferably includes polynucleotide variants. The term "polynucleotide variant", as used herein, relates to a variant of a polynucleotide referred to herein comprising a nucleic acid sequence characterized in that the sequence can be derived from the aforementioned specific nucleic acid sequence by at least one nucleotide substitution, addition and/or deletion, wherein the polynucleotide variant shall have the function and/or activity as specified for the specific polynucleotide. Thus, a variant of a marker sequence may e.g. be a sequence of an ortholog, a paralog, or another homolog of the specific polynucleotide. Also preferably, said polynucleotide variant is or is derived from a non-naturally occurring allele of the specific polynucleotide. Polynucleotide variants also encompass polynucleotides comprising a nucleic acid sequence which is capable of hybridizing to the aforementioned specific polynucleotides, preferably, under stringent hybridization conditions. These stringent conditions are known to the skilled worker and can be found e.g. in Current Protocols in Molecular Biology, John Wiley & Sons, N. Y. (1989), 6.3.1-6.3.6. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. As referred to herein, "hybridizing specifically" preferably means hybridizing under stringent conditions, more preferably means hybridizing to the target sequence compared to a non-target sequence by a factor at least 2fold, preferably at least 5fold, more preferably at least 10fold, even more preferably at least 100fold. Thus, a primer or probe specifically hybridizing to a target sequence provides for a specific amplification and/or a specific signal. Further polynucleotide variants include polynucleotides comprising nucleic acid sequences which are at least 70%, preferably at least 80%, more preferably at least 90%, even more preferably at least 95%, still more preferably at least 98%, most preferably at least 99%, identical to the specifically indicated nucleic acid sequences. The percent identity values are, preferably, calculated over the entire nucleic acid sequence region, preferably as specified herein elsewhere. The polynucleotides of the present invention either consist, essentially consist of, or comprise the aforementioned nucleic acid sequences. Thus, they may contain further nucleic acid sequences as well.

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the sequence, typically, the polynucleotide sequence, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (e.g. BLAST, GAP, BESTFIT, PASTA, or TFASTA), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. More preferably, the Basic Local Alignment Search Tool (BLAST) implementation is used with default parameter values for alignment. In the context of biological sequences referred to herein, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "sample", as used herein, refers to any composition of matter known or suspected to comprise cfDNA, preferably known or suspected to comprise short ds cfDNA to be detected. The sample may be of any state of matter. Thus, preferably, the sample is a liquid, semisolid, solid, or gaseous sample. Preferably, the sample is a liquid or semisolid sample, preferably an aqueous solution, which may optionally comprise other solvents or other biological material. Typically, the sample is derived from a body fluid or is a sample of a body fluid, typically the body fluid of a vertebrate species. All types of vertebrate species are generally envisaged, however, in particular, the sample may relate to a body fluid of a chicken, a goose, a duck, a goat, a sheep, a cattle, a pig, a horse, a dog, a cat, a hamster, a rat, a mouse, a hamster, or a guinea pig or of a human subject. Preferably, the body fluid is from a mammalian species. More preferably, the body fluid is of is a human. In line with the present invention, the sample typically is a sample of a body fluid, preferably selected form the group consisting of blood, plasma, serum, lacrimal fluid, urine, lymph, cerebrospinal fluid, bile, stool, sweat, amniotic fluid, synovial fluid and saliva, more preferably the sample is a plasma sample.

Samples can be obtained by well-known techniques which include, preferably, scrapes, swabs or biopsies. Such samples can be obtained by use of brushes, (cotton) swabs, spatulas, rinse/wash devices, punch biopsy devices, puncture devices for cavities, such as needles or lances, or by other surgical instrumentation. Typically, samples may be obtained from cell culture supernatants, body fluids, or tissues or organs by separating techniques such as filtration, centrifugation, or cell sorting. The sample may in particular be a blood or blood-derived sample as specified herein, a lacrimal fluid, urine, lymph, cerebrospinal fluid, bile, stool, sweat, amniotic fluid, synovial fluid or a saliva sample. A sample in lie with the present invention may typically be referred to as a liquid biopsy sample. A "liquid biopsy sample" typically relates to a sample of a body fluid that can be used for diagnostic purposes replacing a common biopsy sample typically obtained by invasive techniques. The method according to the invention may comprise a step of obtaining a sample from the subject. However, preferably the method according to the invention is an *ex vivo* method that is performed with an isolated sample.

Using a sample as specified herein in the method according to the invention is advantageous as it may typically be obtained by a non-invasive or minimally invasive technique. It hence allows for a minimally invasive but reliable diagnostic measure for a large number of diseases and disorders that up to date require massively invasive diagnostic procedures based on biopsies. It is further suitable for non-invasive prenatal diagnostic approaches (NIPD).

The method according to the invention comprises a step of isolating cfDNA of a sample of a subject, e.g. of a sample of body fluid. It is to be understood that the sample may be further processed in order to carry out a method of the present invention. The method according to the invention is preferably an *ex vivo* method.

cfDNA and methods for its isolation are known in the art, e.g. from Aucamp et al. (2018) Biol. Rev. 93, 1649-1683 and further specified elsewhere herein.

Moreover, in line with the invention cfDNA may be enriched, e.g. by precipitation and/or by binding to a solid surface binding anionic compounds, e.g. magnetic beads binding DNA. Also preferably, cells or other particulate matter may be removed by centrifugation and/or filtration, and the like. Further, a subfraction of cfDNA in a sample may be isolated as cfDNA, e.g. by size fractionation, separation or selection. Preferably, the aforesaid enrichment and/or subfractionation is non-sequence specific. As referred to herein, all types of pretreated samples are included in the term sample.

The term "biomarker", as used herein, generally relates to an indicator of the severity or the presence of a physiological or pathological condition, e.g. a disease or disorder, of an organism. In particular, a "biomarker" according to the present invention is a nucleic acid molecule or a combination of nucleic acid molecules making possible the identification of a physiological condition, disease or disorder of interest. Said identification typically relies on the presence, absence or abundance of the biomarker. Thus, presence, absence or abundance of the nucleic acid molecule or of a combination of nucleic acid molecules is typically unique to the physiological condition, disease or disorder of interest. Hence, the abundance of the biomarker may, however, be indicative for a physiological condition, disease or disorder of interest. A biomarker according to the present invention is typically characterized by a nucleic acid sequence, more typically a genomic sequence, e.g. a genomic sequence of the reference genome. In particular, a biomarker according to the invention can be characterized by a specific target sequence; typically a sequence defined by a core target sequence and flanking sequences upstream and downstream thereof. In line with the present invention, the biomarker is a cfDNA derived biomarker associated with the physiological condition, disease or disorder of interest.

The term "cfDNA-derived biomarker" typically refers to a nucleic acid molecule or a combination of nucleic acid molecules identified by the method according to the invention. In particular, the "cfDNA-derived biomarker" represents a, typically assembled, nucleic acid sequence, of one or several genomic locations of a reference genome. The cfDNA derived biomarker typically corresponds to gene regulatory regions in the reference genome such as transcriptional start sites, transcription factor binding sites, gene promoter regions, CpG islands, and binding site of structural proteins, more typically to transcriptional start sites, transcription factor binding sites and gene promoter regions. The nucleic acid sequence may typically have a length corresponding to a single short cfDNA fragment up to a length of a few hundred bases. Said nucleic acid sequence is typically a contiguous sequence of the reference genome. In case several genomic locations are involved, the cfDNA-derived biomarker may also be referred to as abundance matrix or biomarker signature. More typically, a cfDNA derived biomarker is characterized by a target sequence of at least 20 bp up to 500 bp or even more on the reference genome. The sequence and size of the cfDNA derived biomarker specifically varies dependent on the physiological condition, disease or disorder of interest. In particular, a cfDNA derived biomarker in line with the invention may be characterized by a target sequence defined by a core target sequence of at least 20 bp and flanking sequences upstream and downstream thereof starting from 5 bp on each side up to 200 bp on the reference genome. A preferred size of a cfDNA derived biomarker as discovered in the present invention is between 30 bp and 300 bp.

A cfDNA derived biomarker may typically be characterized by its presence or abundance in a sample of a subject known to be in the physiological condition of interest or to suffer from the disease or disorder of interest, or it may be characterized by its absence from said sample. Typically, a cfDNA derived biomarker according to the present invention corresponds to a differentially enriched region identified by comparing short cfDNA fragments of a sample to a reference, in particular as described elsewhere herein in further detail.

Differentially enriched regions (DERs) can be identified, typically between nucleic acid sequences determined from a sample and a reference, using appropriate software tools known in the art; for example with the R package DEBrowser (v1.2.0) using the implemented edgeR method. Typically, differential enrichment analysis is performed on the nucleic acid sequences determined of the short ds cfDNA fragments, more typically on the raw read counts of consensus peaks as defined elsewhere herein; in particular in sets of consensus peaks. Still more typically, differential enrichment analysis is performed on said nucleic acid sequences of a sample compared to a reference, in particular to nucleic acid sequences of a reference sample. Differential enrichment may typically be determined by comparing said nucleic acid sequences of a sample to nucleic acid sequences of a reference. Nucleic acid sequences are typically considered as differentially enriched between a sample and a reference when the difference is statistically significant. The difference may lie in the presence, absence or the abundance of the compared determined nucleic acid sequences. Typically said sequences are mapped sequences, more typically consensus peaks of mapped sequences as described elsewhere herein.

Whether a value is statistically significant can commonly be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90%, at least 95%. The p-values are, in an embodiment, 0.5, 01, 0.05, or 0.01.

Preferably, nucleic acid sequences are considered as differentially enriched between a sample and a reference with an adjusted p-value smaller than 0.5, and/or a fold change ≤ -1.5 or ≥ 1.5. More typically a p-value smaller than 0.2, even more typically a p-value smaller than 0.1, even more typically a p-value smaller than 0.1 and/or a fold-change ≤ -2 or ≥ 2.

Preferably highly significant DERs relate to the 100 most significant DERs identified by comparing short cfDNA fragments of a sample to a reference, even more typically to the 80 most significant, to the 50 most significant, to the 30, 20, ten or five most significant DERs.

Preferably a combination of combination DERs is used as cfDNA derived biomarker for assessing a physiological condition, disease or disorder of interest as explained elsewhere herein. In complex physiological or pathological condition, using a combination of DERs is advantageous as it is believed to achieve a particularly highly reproducible and reliable diagnostic tool.

The phrase "associated with the physiological condition, disease or disorder of interest" refers to the biomarker being indicative for said physiological condition, disease or disorder of interest. It is to be noted that a biomarker identified in a sample of a pregnant subject may be indicative for a physiological condition, disease or disorder of interest of the fetus.

In particular, the presence, absence or the abundance of a specific biomarker or a combination of biomarkers in a sample is indicative for a physiological condition of interest or a disease or disorder of interest. A biomarker can typically be determined by any means known to the skilled artisan. Specifically, the cfDNA derived biomarker can be identified and/or its presence, absence or abundance can be determined by the methods according to the invention as outlined elsewhere herein.

Preferred biomarkers characterized by DERs are described by reference to the human genome in the Examples below, see Tables 4-7.

The term "physiological condition of interest" as used herein, relates to any type of physiological condition. Typically, the physiological condition of interest may relate to pregnancy, developmental stages of a subject including fetal developmental stages, and agingrelated conditions, training status, aerobic and anaerobic conditions of the body, hormonal conditions including menstrual cycle and insulin status, and further conditions that are typically non-pathological and hence typically do not require medical treatment. In particular, the physiological condition of interest relates to pregnancy or to developmental stages, more particularly the term "physiological condition of interest" may relate to developmental stages, health, growth or nutritional status of the subject, even more particularly of the fetus in a pregnant subject.

Typically, the disease or disorder of interest is a cancer, or an infectious disease. The term "cancer", as used herein, relates to a disease of an animal, including man, characterized by uncontrolled growth by a group of body cells ("cancer cells"). This uncontrolled growth may be accompanied by intrusion into and destruction of surrounding tissue (infiltration) and possibly spread of cancer cells to other locations in the body (metastasis). Preferably, also included by the term cancer is a recurrence of a cancer (relapse). Preferably, the cancer is selected from the list consisting of acute myeloid leukemia (AML), acute lymphoblastic leukemia, adrenocortical carcinoma, aids-related lymphoma, anal cancer, appendix cancer, astrocytoma, atypical teratoid, basal cell carcinoma, bile duct cancer, bladder cancer, bone cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, fibrosarcoma, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, pancreatic ductal adenocarcinoma, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor. More preferably, the cancer is a selected from the list consisting of pancreatic ductal adenocarinoma (PDAC), colorectal carcinoma (CRC), non-small cell lung cancer (NSCLC), bone cancer; even more preferably selected from pancreatic ductal adenocarinoma (PDAC) and colorectal carcinoma (CRC).

The term "infectious disease" or "infection" generally relates to all kinds of infections by pathogenic microorganisms in a subject, including fungal, parasitic, viral or bacterial infections. In line with the present invention, the disease or disorder of interest preferably is an infection by a pathogenic microorganism. Preferably, the infection causes an inflammatory disease, i.e. a health state or pathophysiological condition wherein the body of an infected subject mounts an immune response to a pathogenic microorganism. The infection may be a local infection, e.g. at an infection site, may be tissue or organ specific, e.g. in viral or bacterial infection of a target organ, as e.g. in hepatitis, pancreatitis or encephalitis, or may be generalized, affecting the whole body of a subject as in lyme disease or sepsis. Moreover, the disease or disorder of interest may be a chronic inflammatory disease including chronic inflammatory bowel disease (IBD), such as ulcerative colitis or Crohn's disease; an autoimmune disease, such as multiple sclerosis and rheumatism; or another chronic disease or disorder such as irritable bowel syndrome, metabolic disorder, diabetes, genetic diseases and developmental aberrations.

In line with the present invention, the disease or disorder of interest may particularly be selected from the group consisting of cancer including pancreatic ductal adenocarinoma (PDAC), colorectal carcinoma (CRC), non-small cell lung cancer (NSCLC), bone cancer; fungal, parasitic, viral or bacterial infections including sepsis, pancreatitis, lyme disease, encephalitis; chronic inflammatory diseases including chronic inflammatory bowel disease (IBD) such as ulcerative colitis or Crohn's disease; autoimmune diseases including multiple sclerosis (MS), rheumatism; irritable bowel syndrome; metabolic disorder; genetic diseases or developmental aberrations, particularly genetic diseases or developmental aberrations of the fetus including trisomies.

Preferably, the disease or disorder is a cancer, or an infectious disease, more preferably selected from pancreatic ductal adenocarcinoma (PDAC), colorectal carcinoma (CRC), and sepsis.

The term "subject", as used herein, relates to a vertebrate organism, preferably a livestock or companion animal, such as a chicken, a goose, a duck, a goat, a sheep, a cattle, a pig, a horse, a dog, a cat, a hamster, a rat, a mouse, a hamster, or a guinea pig. More preferably, the subject is a mammal. Even more preferably, the subject is a human. Typically, the subject is known or suspected to suffer from a disease or be in a physiological condition as specified herein above, more typically the subject is known or suspected to suffer from PDAC, CRC or sepsis. The method according to the invention can be applied for non-invasive or minimally invasive prenatal diagnostic approaches. In this case, the term "subject" includes the pregnant mother with the fetus.

The methods according to the present invention comprises a step of isolating cfDNA from a sample containing cfDNA of a subject known to be in the physiological condition of interest or to suffer from the disease or disorder of interest. Methods for the isolation of cfDNA are known in the art, e.g. from Aucamp et al. (2018) Biol. Rev. 93, 1649-1683. These methods may typically rely on silica membrane-based centrifugal force or vacuum extraction. Kits for cfDNA isolation are commercially available for example from Qiagen, Hilden, Germany, or Thermo Fisher Scientific. Typically, the isolation of cfDNA from a sample comprises a separation of cellular components from the sample, more typically followed by protease treatment to remove proteins from the remaining liquid sample. The cfDNA fragments may then be separated from the liquid sample by binding to magnetic beads. In line with the present invention, the isolation of cfDNA preferably refers to the isolation of total cfDNA. In this context, the term "total cfDNA" more preferably refers to cfDNA fragments of all sizes that are isolated in the method according to the invention, typically to the total amount of cfDNA of the isolated double-stranded cfDNA prior to any size selection steps. The step of isolating cfDNA from a sample may also be referred to as preparing cfDNA from a sample thereby generating a preparation of cfDNA that may be processed further.

The method typically further comprises a step of generating a library of the double-stranded cfDNA, preferably a sequencing library of the double-stranded cfDNA.

This may typically be achieved by preparing a double-stranded DNA-specific library; thereby typically selecting for double-stranded cfDNA fragments. Other methods for selecting for ds cfDNA as known in the art, for example non-denaturing polyacrylamide gel electrophoretic separation methods, may be employed. The library is preferably a next generation sequencing library of ds cfDNA. Library generation procedures are known in the art. Kits for these procedures are commercially available such as NEXTFLEX Cell free DNA-Seq Kit (V2) (NOVA-5150-02). In line with the present invention, ds DNA specific primers may be used for generating the sequencing library. Said primers may typically have a size that exceeds the size of the short cfDNA, such as 30 to 100 bp, typically between 50 and 70 bp. The method according to the invention may further comprise obtaining a ds cfDNA library.

Further, the methods according to the present invention comprise a step of selecting short double-stranded cfDNA fragments, typically from the cfDNA preparation. Preferably, the method comprises a step of performing a size selection for short ds cfDNA fragments using any method as known in the art. Specifically, said selecting of short double-stranded cfDNA fragments comprises gel electrophoretic size separation of the double-stranded cfDNA, gradient gel electrophoresis, PCR-based approaches, hybridization and capture, and/or selection using binding protein crosslinking and capture, more specifically gel electrophoretic size separation of a sequencing library of the double-stranded cfDNA.

Still more specifically, gel electrophoretic size separation, gradient gel electrophoresis or PCR-based approaches may be used to select for fragments of up to 100 bp; more typically fragments between 20 bp and 60 bp, even more specifically, gel electrophoretic size separation or gradient gel electrophoresis. Even more typically, size selection of the cfDNA, in particular of the cfDNA library, may be performed using an appropriate preparative gel electrophoresis instrument, specifically with a pulsed-field, such as a BluePippin instrument (Sage Science). Selection of the short ds cfDNA fragments may be performed on an agarose gel, such as a 3% agarose. Other approaches including size-specific solid phase extraction, size-dependent chromatographic separation, and further methods known in the art are possible. Alternatively, a selective enrichment based on known sequences may be performed including sequence directed selection, such as hybridization and capture approaches. Moreover, following size selection, typically a quality check to ensure normal size distribution may be performed. Size selection may comprise a re-amplification step on the size selected sample. Moreover, size selection may be repeated if fragments outside of the selected size were detectable. Further, quality of the size selection may be assessed using appropriate means in the art such as the Fragment Analyzer High Sensitivity DNA Kit (Agilent).

Preferably, selecting short ds cfDNA fragments results in an enrichment of the short ds cfDNA fragments by at least 2-fold, at least 4-fold, at least 10-fold, at least 50-fold, at least 75-fold, or at least 100-fold, preferably as compared to the non-enriched total cfDNA preparation. The enrichment typically refers to an enrichment of at least two-fold or more defined by the ratio of short cfDNA versus regular cfDNA; more preferably short cfDNA refers to cfDNA fragments with a length of at most 100 bp or below. The enrichment of short cfDNA can be determined by quantifying the amount of total cfDNA and the amount of short cfDNA prior and after the enrichment. For example, in a typical ds cfDNA preparation of a healthy subject, the amount of short cfDNA fragments is commonly in the range of 0.2 % - 3 % of total cfDNA (Hudecova et al., 2022). After the size selection as described herein, the amount of short cfDNA fragments is significantly increased, and typically as high as 5 % and up to 99% of total cfDNA in the preparation after size selection. More typically, the size selection results in an amount of short cfDNA fragments at least 50 % of total cfDNA in the preparation after size selection; even more typically of at least 60 %, at least 70 %, at least 80 %, at least, 90 %, at least 95% , at least 96%, at least 97 %, at least 98 %, or at least 99 % of total cfDNA in the preparation after size selection.

The size selection in the method according to the invention is advantageous as it enables the enrichment of the short cfDNA fragments and allows for downstream analysis of short cfDNA fragments with high accuracy but without the need for ultra deep sequencing or high coverage, i.e. 10 x or more.

The method according to the invention, comprises a step of determining the nucleic acid sequences of said short double-stranded cfDNA fragments. The term generally refers to the identification of the nucleic acid sequence of nucleic acid molecules of all sizes, in particular of cfDNA fragments. Means and methods for determining nucleic acid sequences are known in the art. A person of skill in the art is in the position of selecting the appropriate sequencing method. The determination of the nucleic acid sequences encompasses qualitative determination, i.e. the identification of the nucleic acid sequence, as well as quantitative or semi quantitative determination, i.e. determining the amount or relative amount of the nucleic acid sequence.

According to the present invention, nucleic acid sequences are typically determined using next generation sequencing and/or deep sequencing known in the art, preferably using Illumina sequencing. More typically, the sequences are determined with a coverage equivalent to at most 10 x of the reference genome, even more typically with a coverage equivalent to at most 5 x, 2 x, 1 x, 0.5 x, 0.2 x or 0.1 x of the reference genome. A commonly applied coverage according the invention is a maximum of 1 x equivalent to the reference genome. For example, in the examples provided below, only about 30 million reads at 100 bp/ read provided sufficient sequencing depth for samples derived from human DNA, i.e. with the human genome being the reference genome. Methods for calculating coverage, such as the Lander/Waterman equation, are known in the art. In case of a sequence directed selection, the coverage refers to an equivalence to the selected target region.

Preferably, the determined nucleic acid sequences of short ds cfDNA fragments comprise protein binding footprint DNA sequences including protein-DNA interaction sites such as transcription factor (TF) binding sites, binding sites of structural proteins, binding site of regulatory proteins, transcriptional starts sites, or gene promoter sequences, CpG islands, preferably TF binding sites. Without wishing to be bound by theory, it is thought by the present inventors that the interaction with or binding to a protein prevents the short ds cfDNA fragments from further fragmentation and/or complete degradation.

The term "reference" as used in accordance with the present invention relates to a reference sample or any amount (reference amount) or value (reference value) which by comparison to the determined nucleic acid sequences of said short double-stranded cfDNA fragments allows for identifying a cfDNA derived biomarker for a physiological condition, disease or disorder of interest, or for assessing a physiological condition, disease or disorder of interest in a sample of a subject suspected to suffer therefrom. Thus, the reference sample or reference amount or reference value may be obtained from a healthy subject.

Preferably, the reference according to the invention comprises one or more short double-stranded cfDNA fragments that are (i) derived from a sample of a body fluid from a healthy subject or a sample of a body fluid from a subject not being in the physiological condition of interest or not suffering from the disease or disorder of interest, or (ii) are known short double-stranded cfDNA fragments.

The "reference sample" according to the invention is preferably a preparation of short double stranded cfDNA fragments of a sample from a healthy subject of the same species, or synthetically produced solution of short ds cfDNA fragments. In particular, the reference sample may be a sample of a body fluid or tissue of a healthy subject of the same species. The term "synthetically produced solution of short ds cfDNA fragments" covers any type of aqueous solution to which known short ds cfDNA fragments have been added and/or an aqueous solution known to contain a predetermined amount of known short ds cfDNA fragments. According to the invention, the reference sample is particularly preferably a sample from a healthy subject, more particularly the reference sample is a sample of a healthy subject that is has recently been operated on, i.e. that is in a postoperative condition, such as a subject that has recently undergone surgery; wherein "recently" typically refers to a few minutes to a few hours post surgery to 2 days post operation

In the method for identifying a cfDNA derived biomarker according to the present invention, the reference preferably is a reference sample. A "healthy subject" is preferably a subject, who is apparently healthy and/or is known not to be in the condition of interest and/or not to suffer from the disease or disorder of interest. In particular, the healthy subject does not suffer from the disease or disorder of interest. The term "healthy subject" in line with the present invention preferably includes post-operative subjects and subjects suffering from any disease or disorder other than the disease or disorder of interest including cancer.

The terms "reference amount" or "reference value" according to the invention may typically refer to an amount or value indicating the abundance of at least one short ds cfDNA fragment, preferably a specific cfDNA fragment. More typically, the term "reference amount" or "reference value" may indicate an average amount of value for the abundance of any short ds cfDNA fragment. In particular, in the method for assessing a physiological condition, disease or disorder of interest in a sample of a subject according to the invention, the reference may relate to a "reference amount" or "reference value".

The term "comparing to a reference", according to the present invention, typically refers to any comparison of a sample to a reference suitable for identifying a cfDNA derived biomarker for a physiological condition, disease or disorder of interest. More typically, any known statistical measure to infer discriminatory power or difference of mean/median or difference of distributions may be applied for said comparison. Among these statistical measures are for example differential analysis using generalized linear model with likelihood ratio test, t-test, Wilcoxon rank sum test, Anova, Kolmogorov Smirnov test, Wald test, Receiver operating characteristic, effect size, mutual information, feature importance in machine learning models (e.g. random forests). Still more typically "comparing to a reference" refers to differential enrichment analysis as describes elsewhere herein.

According to the invention, preferably at least one short double-stranded cfDNA fragment or a multitude of short double-stranded cfDNA fragments, which is/are absent from short double-stranded cfDNA fragments of the reference, or which differ/s in abundance thereto, is/are identified as at least one cfDNA derived biomarker associated with the physiological condition, disease or disorder of interest. In line with the invention, the phrase "differs in abundance to the short ds cfDNA fragments of the reference" includes absence of the short ds cfDNA fragment from the sample. Preferably, for the identification of the cfDNA derived biomarker associated with the physiological condition, disease or disorder of interest the method of the invention is repeated for a multitude of samples of different subjects known to be in the same physiological condition of interest, or known to suffer from the same disease or disorder of interest, preferably for a multitude of biological replicates. More preferably, the repetition may take place in parallel such as that multiple samples are processed at the same time.

The method further comprises a step of mapping the determined nucleic acid sequences of the short double-stranded cfDNA fragments to a reference genome, preferably thereby obtaining mapped nucleic acid sequences, also referred to as mapped sequences herein. Mapping of the determined nucleic acid sequence to the reference genome may serve as a suitable tool to identify the origin of the cfDNA fragments, in other words to identify the position of the cfDNA fragment on the reference genome. The mapped sequences may typically be aligned to, preferably correspond to, gene regulatory regions in the reference genome such as transcriptional start sites, transcription factor binding sites, gene promoter regions, CpG islands, and binding site of structural proteins, more typically to transcriptional start sites, transcription factor binding sites and gene promoter regions. Means and methods for mapping nucleic acid sequences to a reference genome, i.e. a genome with a known assembled sequence, for example the human reference genome assembly GRCh37, are known in the art and readily available to the skilled artisan.

Preferably, the reference genome is a vertebrate genome, preferably a mammalian genome, more preferably a human, a goat, a sheep, a cattle, a pig, a horse, a dog, a cat, a hamster, a rat, a mouse, a hamster, or a guinea pig genome, still more preferably the human genome. The reference genome typically is a genome with a known assembled sequence.

According to the present invention, the step of mapping the determined nucleic acid sequence may typically be performed using available software tools known to the person of skill in the art such as NextGenMap (v0.5.5), or any other short read alignment or mapping tool known in the art including Bowtie, BWA, HISAT, STAR.

Moreover, prior to mapping, the method may comprise at least one or more of the following steps: sequencing quality control, sequence adapter removal, removing terminal poly G sequences, quality trimming, removing sequencing reads with low complexity. Typically, the mapped sequences are processed using appropriate software tools such as SAMtools by Heng Li, available at www.htslib.org. The processing of the mapped sequences may include at least one or more of the following steps: deduplication; filtering blacklisted regions, MapQ filtering; deep tool downstream analyses as generally known in the art.

The mapped sequences may typically serve as a basis for identifying consensus peaks. The term "consensus peaks" as used herein typically represent a multitude of mapped sequences within the same position of the reference genome identified in a sample of a subject known to be in the physiological condition of interest or to suffer from the disease or disorder of interest. Consensus peaks may typically be established across more than one sample of different subjects known to be in the physiological condition of interest or to suffer from the disease or disorder of interest. Consensus peaks may be narrow, such as between 20 bp and 100 bp or wide, such as between 101 bp and 500 bp. Typically consensus peaks are defined as genomic locations, in particular where a narrow peak is identified in at least three of four samples; more particularly, said samples refer to samples containing cfDNA of subjects known to be in the physiological condition of interest or to suffer from the disease or disorder of interest. Consensus peaks may typically be derived from mapped sequences using appropriate bioinformatics tools known to the skilled artisan such as MACS2. Consensus peaks associated with a physiological condition, disease or disorder of interest preferably are identified using appropriate bioinformatics tools such as R known in the art.

In the method according to the invention of identifying a biomarker, steps a) to e) may be repeated, typically on a sample of a different subject known to be in the physiological condition of interest or to suffer from the disease or disorder of interest; more typically thereby verifying the biomarker.

Preferably, in line with the present invention, the comparison in step d) comprises comparing the mapped sequences of the short double-stranded cfDNA fragments to the mapped sequences of the short double-stranded cfDNA fragments of the reference, in particular of the reference sample. More preferably, the method further comprises a step of deriving consensus peaks from the mapped sequences. Still more preferably, the method comprises comparing the consensus peaks derived from the mapped sequences of the short double-stranded cfDNA fragments of the sample to the consensus peaks derived from the mapped sequences of the short double-stranded cfDNA fragments of the reference. The comparison typically comprises differential enrichment analysis and hence may more typically result in the identification of differentially enriched regions between short ds cfDNA fragments of the sample and of the reference. In other words, the comparison may typically comprise, or consist of, a differential enrichment analysis of consensus peaks derived from the mapped sequences of the short double-stranded cfDNA fragments of sample and reference. Preferably, the method for identifying a cfDNA derived biomarker according to the invention comprises identifying differentially enriched regions (DERs). In line with the present invention, the method for identifying a cfDNA derived biomarker comprises step d) of comparing the nucleic acid sequences of said short double-stranded cfDNA fragments to a reference; typically thereby differentially enriched regions (DERs) are identified.

Moreover, in the method for identifying a cfDNA derived biomarker according to the invention, the at least one disease or disorder associated cfDNA derived biomarker is preferably based on a combination of more than one DER.

The definitions made above apply mutatis mutandis to the following. Additional definitions and explanations made further below also apply for all embodiments described in this specification mutatis mutandis. In particular, definitions and explanations made with respect to the method steps explained herein above may applied to the steps of the methods below unless otherwise specified .

Further the present invention relates to a method for assessing a physiological condition, disease or disorder of interest in a sample of a subject suspected to be in said condition or to suffer from said disease or disorder of interest, comprising: a) isolating cfDNA from a sample containing cfDNA of said subject; b) selecting short double-stranded cfDNA fragments; c) determining the nucleic acid sequences of said short double-stranded cfDNA fragments; and d) determining the presence, absence or abundance of at least one cfDNA derived biomarker associated with the physiological condition, disease or disorder of interest within the determined nucleic acid sequences of said short double-stranded cfDNA fragments.

The term "determining the presence, absence or abundance of at least one cfDNA derived biomarker", as used herein, refers to each and every measure for determining the presence, absence, or amount of a cfDNA derived biomarker in a sample. Thus, the term typically encompasses qualitative determination, e.g. determination of the presence of a cfDNA derived biomarker sequence above a pre-determined detection limit, as well as quantitative or semi quantitative determination i.e. determining the amount or relative amount of the cfDNA derived biomarker. The terms "abundance" and "amount" are used interchangeably herein. The determination is based on the determination of the nucleic acid sequences in step c). The determination preferably comprises selecting from the determined nucleic acid sequences the target sequence and determining the abundance thereof.

Preferably, in the method for assessing a physiological condition, disease or disorder of interest according to the invention, the at least one cfDNA derived biomarker associated with the physiological condition, disease or disorder of interest is identified or identifiable by the method for identifying a biomarker as described elsewhere herein.

The method for assessing a physiological condition, disease or disorder of interest according to the invention may further comprise comparing the determined abundances of the at least one cfDNA derived biomarker associated with the physiological condition disease, or disorder of interest to a reference.

Typically, said method for assessing a physiological condition, disease or disorder of interest according to the invention further comprises a step of assessing the condition of interest, disease or disorder in a sample of a subject.

The term "assessing" as used herein refers to establishing a physiological condition of interest, or diagnosing a disease or disorder of interest in a subject. As will be understood by those skilled in the art, an assessment is usually not intended to be correct for 100% of the subjects to be investigated. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

More specifically, the method of the present invention when assessing a physiological condition, disease or disorder of interest, comprises identifying whether the subject is in the physiological condition of interest or suffers from the disease or disorder of interest.

The present invention furthermore contemplates a method for determining efficacy of treating a physiological condition, disease or disorder of interest in a sample of a subject known to be in said condition or suffer from said disease or disorder comprising: a) isolating cfDNA from a sample containing cfDNA of said subject; b) selecting short double-stranded cfDNA fragments; c) determining the nucleic acid sequences of said short double-stranded cfDNA fragments; d) determining the presence, absence or abundance of at least one cfDNA derived biomarker associated with the physiological condition, disease, or disorder of interest within the determined nucleic acid sequences of said short double-stranded cfDNA fragments; e) repeating steps a) to d); and f) determining efficacy of therapy based on the determination over time.

The step of "determining efficacy of therapy based on the determination over time" as used herein, refers to each and every measure for determining the success of treatment over time. Typically, the determination involves a comparison of a sample of a subject known to be in said condition or to suffer from said disease or disorder after treatment with a sample of a subject known to be in said condition or to suffer from said disease or disorder prior to or without treatment. The term "after treatment" may typically refer to an appropriate point in time, or typically more than one point in time, after appropriate treatment has started and at which an effect is typically to be expected.

The method may further comprise a step of determining a treatment response. In particular, determining a success of the treatment if improvement of the physiological condition or disease or disorder in the subject upon treatment occurs or determining a failure of the treatment if worsening of the physiological condition or disease or disorder occurs in the subject upon treatment, or if the physiological condition, disease or disorder remains unchanged.

In the method for determining efficacy of treating a physiological condition, disease or disorder of interest steps a) to d) are preferably repeated at a suitable interval; more preferably, at least once every year to several times a week; even more preferably at least once every four months to at least ten times per month.

Moreover, the present invention relates to a device for detecting at least one cfDNA derived biomarker in a liquid biopsy sample comprising at least: 1) a sample collector and selector for isolating and selecting cfDNA; 2) a sequencer, preferably, for high-throughput sequencing; and 3) an evaluating unit comprising at least a processor. The sample collector and selector is typically a unit of the device adapted to receive, typically to obtain, cfDNA from a sample, preferably from a plurality of samples, of a subject. More typically, the sample collector and selector may isolate the cfDNA from the samples by an automated cfDNA preparation procedure and may specifically select for short ds cfDNA. Moreover, the sample collector and selector may typically prepare, the cfDNA preparations for further automated processing in the sequencer. The sequencer may be any sequencer known in the art suitable for sequencing of short ds cfDNA, typically it may be a next generation sequencing device as known in the art. The evaluating unit comprises at least a processor, typically a microprocessor, comprising tangibly embedded executable instructions for performing at least one step of a method as specified herein. Preferably the evaluating unit further comprises a storage medium. The sample collector and selector, the sequencer and evaluating unit are typically assembled into a functional unit.

Preferably, the device is adapted to perform the methods according to the invention, in particular, the method for identifying a cfDNA derived biomarker for a physiological condition, disease or disorder of interest, and/or the method for assessing a physiological condition, disease or disorder of interest according to the invention, and/or the method for determining efficacy of treating a physiological condition, disease or disorder of interest described elsewhere herein.

The present invention moreover contemplates a cfDNA derived biomarker for use in diagnosing a disease or disorder in a subject suspected to suffer therefrom. Preferably, said cfDNA derived biomarker is obtained and/or is obtainable by the method for identifying a cfDNA derived biomarker as disclosed herein.

In view of the above, the following embodiments are particularly envisaged:
1. A method for identifying a cfDNA derived biomarker for a physiological condition, disease or disorder of interest, comprising:
   a) isolating cfDNA from a sample containing cfDNA of a subject known to be in the physiological condition of interest or to suffer from the disease or disorder of interest;
   b) selecting short double-stranded cfDNA fragments;
   c) determining the nucleic acid sequences of said short double-stranded cfDNA fragments;
   d) comparing the nucleic acid sequences of said short double-stranded cfDNA fragments to a reference; and;
   e) based on said comparison in step d) identifying at least one cfDNA derived biomarker associated with the physiological condition, disease or disorder of interest.
2. The method of embodiment 1, wherein the short double-stranded cfDNA fragments are double-stranded DNA fragments with a fragment size below 100 bp, preferably between 10 and 80 bp, more preferably between 20 and 60 bp.
3. The method of embodiment 1 or 2, wherein the sample is a sample of a body fluid, preferably selected form the group consisting of: blood, plasma, serum, lacrimal fluid, urine, lymph, cerebrospinal fluid, bile, stool, sweat, amniotic fluid, synovial fluid and saliva, more preferably the sample is a plasma sample.
4. The method of any of embodiments 1 to 3, wherein the selecting in step b) results in an enrichment of the short ds cfDNA fragments by at least 2-fold, at least 4-fold, at least 10-fold, at least 50-fold, at least 75-fold, or at least 100-fold, preferably wherein enrichment refers to an enrichment of at least two-fold defined by the ratio of short cfDNA versus regular cfDNA; more preferably wherein short cfDNA refers to cfDNA with a length of at most 100 bp or below.
5. The method of any of embodiments 1 to 4, further comprising a step of generating a library of the double-stranded cfDNA, preferably a sequencing library of the double-stranded cfDNA.
6. The method of any of embodiments 1 to 5, wherein said selecting short double-stranded cfDNA fragments comprises gel electrophoretic size separation of the double-stranded cfDNA, gradient gel electrophoresis, PCR-based approaches, hybridization and capture, and/or selection using binding protein crosslinking and capture, preferably gel electrophoretic size separation of a sequencing library of the double-stranded cfDNA.
7. The method of any of embodiments 1 to 6, wherein the sequences are determined using next generation sequencing, preferably using Illumina sequencing.
8. The method of any of embodiments 1 to 7, wherein the determined nucleic acid sequences comprise protein binding footprint DNA sequences including protein-DNA interaction sites such as transcription factor (TF) binding sites, binding sites of structural proteins, binding site of regulatory proteins, transcriptional start sites, or gene promoter regions, CpG islands, preferably TF binding sites.
9. The method of any of embodiments 1 to 8, wherein steps a) to e) are repeated, typically on a sample of a different subject known to be in the physiological condition of interest or to suffer from the disease or disorder of interest; more typically thereby verifying the biomarker.
10. The method as in embodiment 8, wherein the abundance and/or the type of protein binding footprint DNA sequences in the determined nucleic acid sequences of the short double-stranded cfDNA fragments differ in comparison to the reference.
10. The method of any of embodiments 1 to 9, wherein the reference comprises one or more short double-stranded cfDNA fragments that are (i) derived from a sample of a body fluid from a healthy subject or a sample of a body fluid from a subject not being in the physiological condition of interest or not suffering from the disease or disorder of interest, or (ii) are known short double-stranded cfDNA fragments.
11. The method of any of embodiments 1 to 10, wherein at least one short double-stranded cfDNA fragment or a multitude of short double-stranded cfDNA fragments, each having a nucleic acid sequence which is absent from short double-stranded cfDNA fragments of the reference or which differs in abundance thereto, is identified as at least one cfDNA derived biomarker associated with the physiological condition, disease or disorder of interest.
12. The method of any of embodiments 1 to 11, wherein the physiological condition, disease or disorder of interest is selected from the group consisting of:
   i) cancer including pancreatic ductal adenocarcinoma (PDAC), colorectal carcinoma (CRC), non-small cell lung cancer (NSCLC), bone cancer;
   ii) fungal, parasitic, viral or bacterial infections including sepsis, pancreatitis, lyme disease, encephalitis;
   iii) chronic inflammatory diseases including chronic inflammatory bowel disease (IBD) such as ulcerative colitis or Crohn's disease;
   iv) autoimmune diseases including multiple sclerosis (MS), rheumatism;
   v) metabolic disorder;
   vi) irritable bowel syndrome;
   vii) genetic diseases or developmental aberrations in particular of the fetus including trisomies; and
   viii) pregnancy, developmental stages.
   preferably wherein the disease or disorder is a cancer, or an infectious disease, more preferably selected from pancreatic ductal adenocarinoma (PDAC), colorectal carcinoma (CRC), and sepsis.
13. The method of any of embodiments 1 to 12, further comprising a step of mapping the determined nucleic acid sequences of the short double-stranded cfDNA fragments to a reference genome, preferably thereby obtaining mapped sequences.
14. The method of any of embodiments 1 to 13, comprising identifying differentially enriched regions (DERs) typically by said comparison of the nucleic acid sequences of said short double-stranded cfDNA fragments of a sample to a reference.
15. The method of embodiment 13, wherein the reference genome is a vertebrate genome, preferably a mammalian genome, more preferably a human, a goat, a sheep, a cattle, a pig, a horse, a dog, a cat, a hamster, a rat, a mouse, a hamster, or a guinea pig genome, still more preferably the human genome.
16. The method of any of embodiments 13 to 15, wherein the comparison in step d) comprises comparing the mapped sequences of the short double-stranded cfDNA fragments to the mapped sequences of the short double-stranded cfDNA fragments of the reference.
17. The method of any of embodiments 14 to 16, wherein the at least one disease or disorder associated cfDNA derived biomarker is based on a combination of more than one of the DERs.
18. A method for assessing a physiological condition, disease or disorder of interest in a sample of a subject suspected to be in said condition or to suffer from said disease or disorder of interest, comprising:
   a) isolating cfDNA from a sample containing cfDNA of said subject;
   b) selecting short double-stranded cfDNA fragments;
   c) determining the nucleic acid sequences of said short double-stranded cfDNA fragments; and
   d) determining the presence, absence or abundance of at least one cfDNA derived biomarker associated with the physiological condition, disease or disorder of interest within the determined of the nucleic acid sequences of said short double-stranded cfDNA fragments.
19. The method of embodiment 18, wherein the at least one cfDNA derived biomarker associated with the physiological condition, disease or disorder of interest is identified or is identifiable by the method as claimed in any one of claims 1 to 17.
20. The method of any of embodiments 18 or 19, further comprising comparing the determined abundances of the at least one cfDNA derived biomarker associated with the physiological condition disease, or disorder of interest to a reference.
21. The method of any of embodiments 18 to 20, further comprising the step of assessing the condition of interest, disease or disorder in a sample of a subject.
22. A method for determining efficacy of treating a physiological condition, disease or disorder of interest in a sample of a subject known to be in said condition or suffer from said disease or disorder comprising:
   a) isolating cfDNA from a sample containing cfDNA of said subject;
   b) selecting short double-stranded cfDNA fragments;
   c) determining the nucleic acid sequences of said short double-stranded cfDNA fragments;
   d) determining the presence, absence or abundance of at least one cfDNA derived biomarker associated with the physiological condition disease, or disorder of interest within the determined nucleic acid sequences of said short double-stranded cfDNA fragments;
   e) repeating steps a) to d); and
   f) determining efficacy of therapy based on the determination over time.
23. The method of embodiment 22, wherein steps a) to d) are repeated at least once every year to several times a week.
24. A device for detecting at least one cfDNA derived biomarker in a liquid biopsy sample comprising at least:
   1) a sample collector and selector for isolating and selecting cfDNA;
   2) a sequencer, preferably, for high-throughput sequence; and
   3) an evaluating unit comprising at least a processor.
25. The device of embodiment 24, wherein the device is adapted to perform the method as claimed in any of claims 1 to 23.
26. Use of a cfDNA derived biomarker, preferably obtained by the method of any one of claims 1 to 17, in a sample for assessing a disease or disorder in a subject suspected to suffer therefrom.
27. A cfDNA derived biomarker, preferably obtained by the method of any one of embodiments 1 to 17, for use in diagnosing a disease or disorder in a subject suspected to suffer therefrom.
28. A method for diagnosing a disease or disorder in a subject suspected to suffer therefrom, said method comprising:
   (I) determining the presence, absence or abundance of at least one cfDNA derived biomarker associated with the disease, or disorder of interest within the determined nucleic acid sequences of said short double-stranded cfDNA fragments
   (II) based on the determining in step (I) diagnosing said disease or disorder.

All references cited in this specification are herewith incorporated by reference with respect to their entire disclosure content and the disclosure content specifically mentioned in this specification.

### Figure Legends

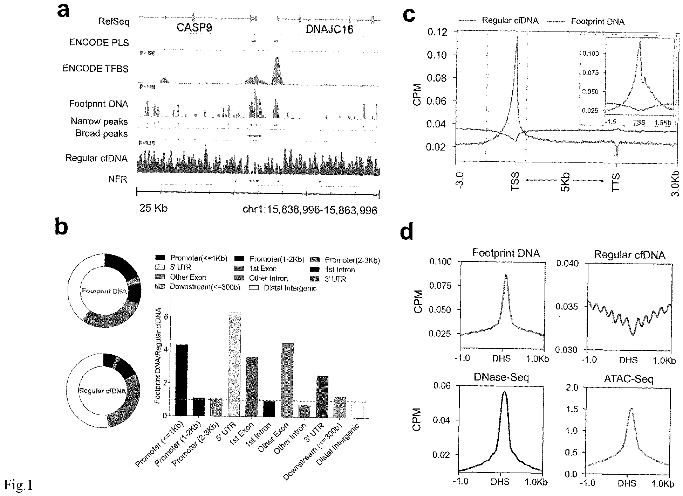

**Fig. 1****: Footprint DNA is enriched in regulatory regions of genes and open chromatin. (a)** A visual example of footprint DNA (S03) in comparison to regular cfDNA (average of S05-S08). For footprint DNA (green), narrow and broad peaks are shown, while nucleosome-free regions (NFRs) are shown for regular cfDNA (violet). RefSeq genes, ENCODE promoter-like structures (PLS), and ENCODE transcription factor binding sites (TFBS) based on ChIP-Seq experiments are included as references. **(b)** Pie charts display the proportions of annotated genomic features for broad peaks from footprint DNA (S03) and NFRs from regular cfDNA (S05-S08 merged). The bar plot shows the ratio between broad peaks and NFRs for each genomic feature. **(c)** Average coverage profiles for footprint DNA (S03) and regular cfDNA (S06) along all annotated protein-coding genes. The gene bodies of all genes are scaled to 5 kilobases. Dashed vertical lines indicate the interval shown in the subplot. The subplot shows average profiles at the transcription start site without re-scaling of the gene body. **(d)** Average coverage profiles for footprint DNA (S03), regular cfDNA (S06), and publicly available ATAC-seq data at DNase hypersensitive sites (DHS) derived from publicly available DNase-seq data. DNase-seq and ATAC-seq data were generated for the GM12878 cell line.

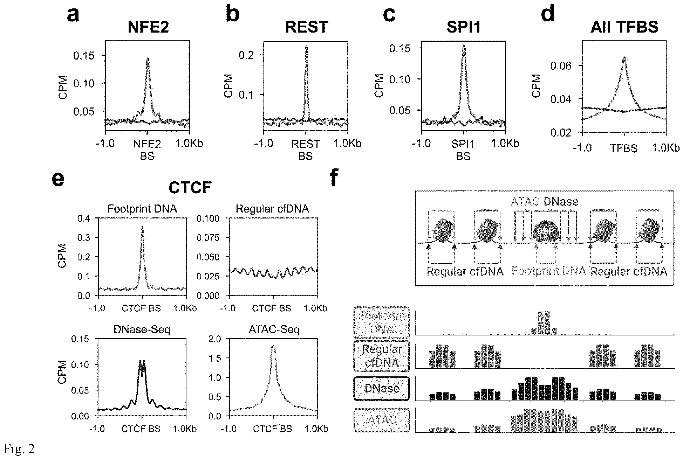

**Fig. 2****: Liquid Footprinting captures the binding of various transcription factors in a direct mechanism. (a-e)** Average coverage profiles of footprint DNA (S03) and regular cfDNA (S06) are shown for three transcription factors (NFE2, REST, and SPI1) from three different transcription factor superclasses. Average profiles are based on the 1000 most robust binding sites annotated in the Gene Transcription Regulation Database (GTRD). In addition, the average profile of all ChIP-seq TFBS annotated in ENCODE3 is shown for footprint DNA in comparison to regular cfDNA. Further average coverage profiles of footprint DNase-seq, and ATAC-seq at CTCF binding sites (CTCF BS) is shown. DNase-seq and ATAC-seq data were generated for the GM12878 cell line. **(f)** Inferred molecular model for the formation of footprint DNA at the binding site of a DNA-binding protein (DBP) surrounded by two nucleosomes on either side. Arrows indicate the endpoints of DNA fragments for the respective sequencing technique. The resulting theoretical coverage tracks are depicted for each sequencing technique.

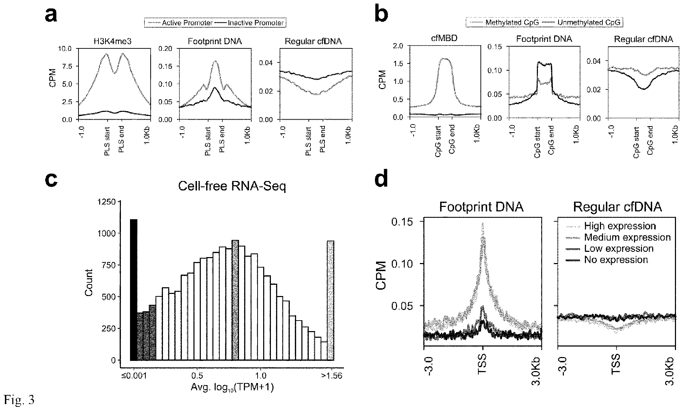

**Fig. 3****: Footprint DNA is enriched to loci with markers of epigenetic activation and transcriptionally active regions. (a)** Annotated promoter-like structures (PLS) from ENCODE are clustered based on publicly available cell-free H3K4me3 ChIP-Seq signal strength into two clusters, representing active (grey line) - or inactive promoters (black line). Average coverage profiles for footprint DNA and normal cfDNA at active or inactive promoters demonstrate the influence of the promoter activation status, **(b)** Annotated CpG islands are clustered based on cell-free Methyl-CpG-Binding Domain (cfMBD) sequencing signal strength into two clusters. Average coverage profiles for footprint DNA and regular cfDNA at methylated (grey line) - or unmethylated (black line) CpG islands reveal the influence of methylation levels at CpG islands. **(c)** Histogram showing average expression levels of protein-coding genes in publicly available cell-free RNA sequencing data. For each category 938 genes were selected (5 % of all analyzed genes): no expression (black), low expression (dark grey), medium expression (medium grey), and high expression (light grey). **(d)** Average coverage profiles for footprint DNA and regular cfDNA at the transcription start sites of the defined gene groups of (c) reveal the influence of transcriptional activity. All data were generated from samples of healthy individuals. S03 was used as the footprint DNA dataset and S06 was used as the regular cfDNA dataset.

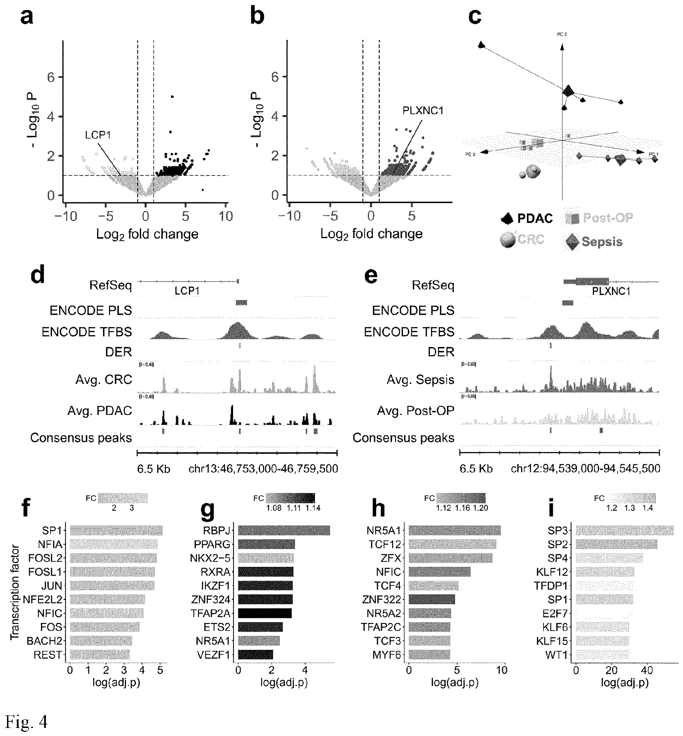

**Fig. 4****: Comparison of liquid footprint data reveals condition-specific transcription factor binding sites and transcription factors, (a)** Differential enrichment analysis for pancreatic ductal adenocarcinoma (PDAC) samples with colorectal cancer (CRC) samples identifies differentially occupied transcription factor binding sites (Adj. p-value ≤ 0.1 and |FC| ≥ 2). **(b)** Differential enrichment analysis for sepsis samples with post-operative (Post-OP) samples identifies differentially occupied transcription factor binding sites (Adj. p-value ≤ 0.1 and |FC| ≥ 2). **(c)** Principal component analysis based on identified differential transcription factor binding sites separates all conditions. Four biological replicates were used per condition. In addition to the samples, a centroid for each group is depicted as a larger data point. Variance explained: PC1 = 29.6 %, PC2 = 20.9 %, PC3 = 8.6 %. **(d)** Example for a differentially enriched TFBS in CRC with novel TF binding in the promoter of LCP1. **(e)** Example for a differentially enriched TFBS in sepsis with novel TF binding in the promoter of PLXNC1. **(f)** - **(i)** Top 10 differentially enriched TF motifs in: **(f)** CRC consensus peaks in comparison to PDAC consensus peaks. CTCF and CTCFL were identified as well but were not included in the figure. **(g)** PDAC consensus peaks in comparison to CRC consensus peaks. **(h)** Sepsis consensus peaks in comparison to Post-OP consensus peaks. **(i)** Post-OP consensus peaks in comparison to sepsis consensus peaks. CTCF and CTCFL were identified as well but were not included in the figure (see Tables 4-7).

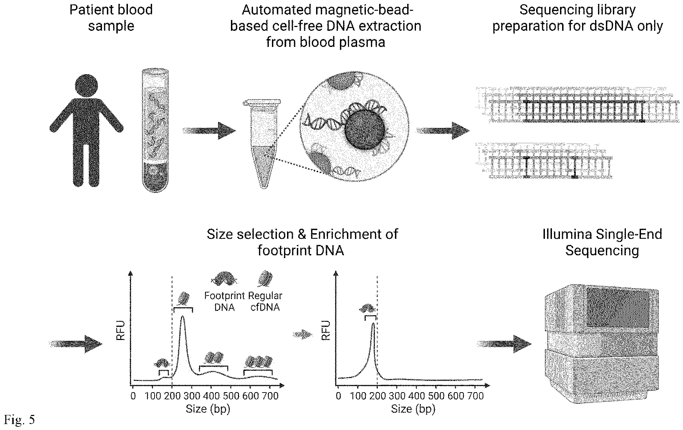

**Fig. 5****: Visual summary of footprint DNA extraction and sequencing from blood plasma.** Cell-free DNA was extracted from the blood plasma of patients using an automated magnetic bead-based kit. Sequencing library preparation was performed by fragment end-repair and adapter ligation. Only intact double-stranded DNA fragments are enriched in the final sequencing library because of the PCR amplification. Footprint DNA is enriched by size selection from sequencing libraries. Footprint DNA sequencing libraries are sequenced on an Illumina platform in single-end mode. This figure was created with biorender.

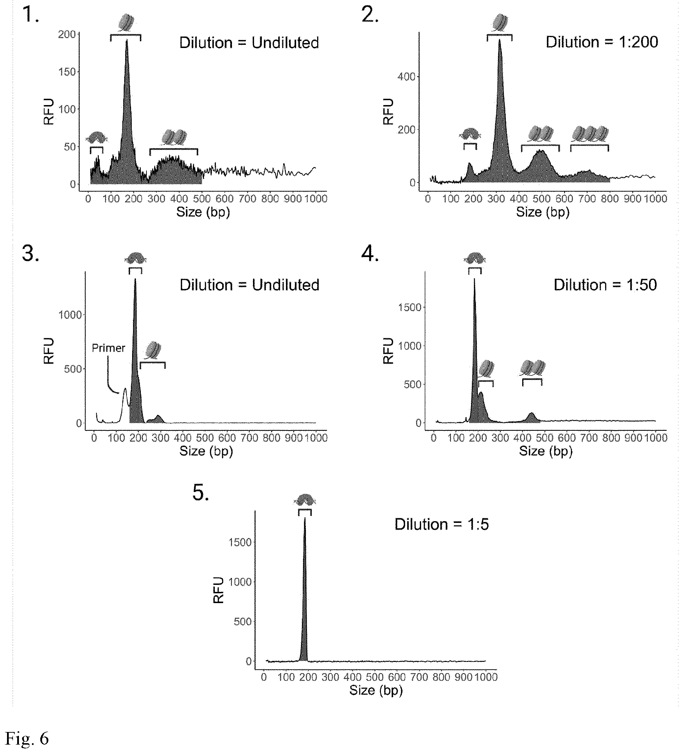

**Fig. 6****: Depiction of the full footprint DNA enrichment process from cell-free DNA for next-generation sequencing.** The steps shown correspond to: **1.** Isolated cell-free DNA. **2.** Double-stranded DNA sequencing library. **3.** Size-selected sequencing library. **4.** PCR-amplified sequencing library. **5.** Sequencing library after second size selection. Size selection was performed using an automated preparative gel electrophoresis instrument (selection window: 150 bp to 200 bp). The applied methods are described in detail in the Methods section. For each step, the Fragment Analyzer profile of S19 and the dilution factor are shown. The purple color indicates DNA fragments that can be assigned to nucleosomes or regular cfDNA, while the green color indicates DNA fragments that can be assigned to footprint DNA.

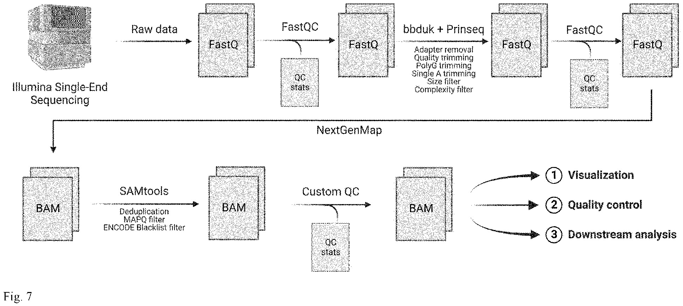

**Fig. 7****: Visual summary of the footprint DNA data processing pipeline.** In short, the raw footprint DNA sequencing data is cleaned and filtered, mapped to a human reference genome, and filtered again before further downstream analysis. The individual steps are described in detail in example 8.

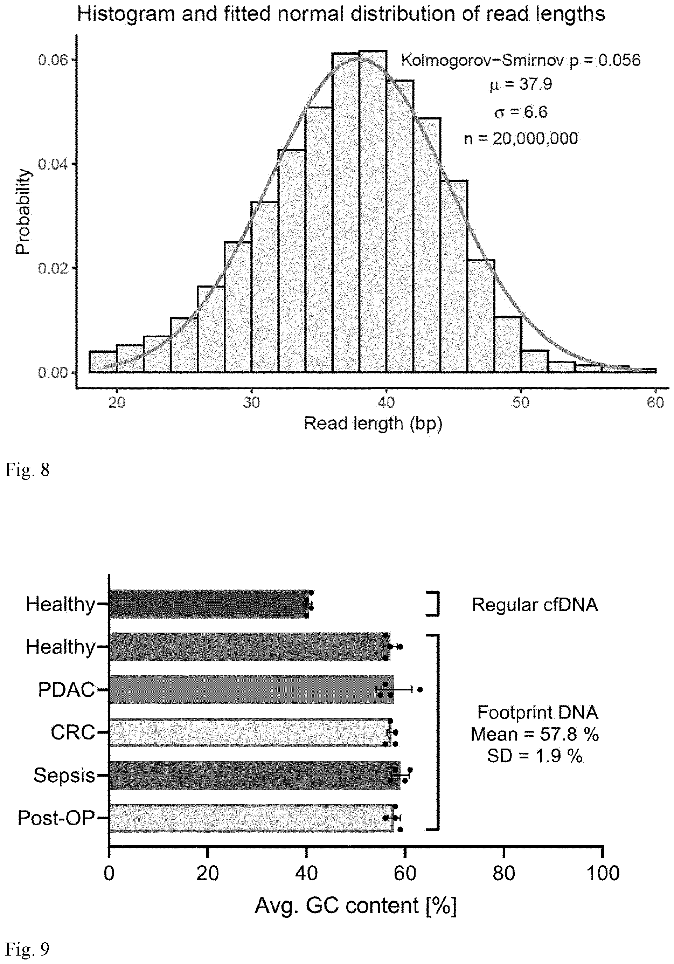

**Fig. 8****: Histogram and fitted normal distribution depicting the observed length of fully processed footprint DNA sequencing reads.** One million random reads were taken from all twenty sequenced liquid footprint samples and their read lengths were plotted in a histogram (total n = 20 million). The observed distribution was fitted with a normal distribution represented by the red line (mean (µ) = 37.9, standard deviation (σ) = 6.6). The fit of the normal distribution to the given data was assessed using a Kolmogorov-Smirnov test, which yielded a p-value of 0.056.
**Fig. 9****: - Average GC content of processed sequencing reads for regular cfDNA and footprint DNA sequencing.** Data from the samples S01 - S24 were used for this analysis (see Table 1). The bar lengths represent the mean value, while error bars indicate standard deviations.

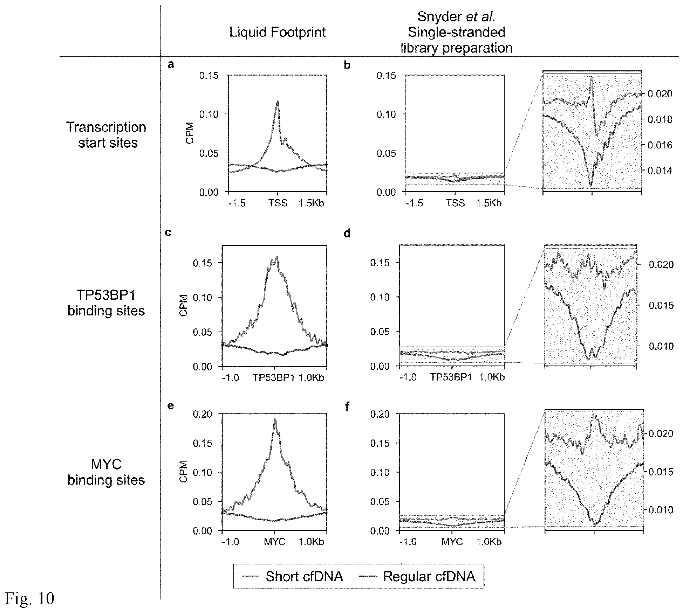

**Fig. 10****: Comparison of liquid footprinting data to single-stranded cfDNA sequencing data from Snyder et al (2016). (a, c, e)** Average coverage profiles based on liquid footprint sequencing data (Sequencing depths: Short cfDNA (S03) = 8.29·10⁶, Regular cfDNA (S06) = 2.60·10⁷). (b, d, f) Average coverage profiles based on sequencing data from Snyder *et al.* generated with a single-stranded library preparation method (Sequencing depths: Short cfDNA = 1.33·10⁸, Regular cfDNA = 3.84·10⁸). These average coverage profiles are also plotted on a smaller scale to better visualize the dynamics of the data. **(a and b)** Average coverage profiles of annotated transcription start sites for short cfDNA and regular cfDNA. **(c and d)** Average coverage profiles of one thousand ChIP-Seq validated TP53BP1 binding sites for short cfDNA and regular cfDNA. **(e** - **f)** Average coverage profiles of one thousand ChIP-Seq validated MYC binding sites for short cfDNA and regular cfDNA. The purple color indicates data from regular cfDNA, while the green color indicates data from short cfDNA. Ultra-deep sequencing data by Snyder *et al.* was produced from the blood plasma of a healthy individual with a single-stranded library preparation method. Raw sequencing data were retrieved from SRA (file ID = SRR2130051) and split into short cfDNA (35-80 nt) and regular cfDNA (120-180 nt) *in silico.*

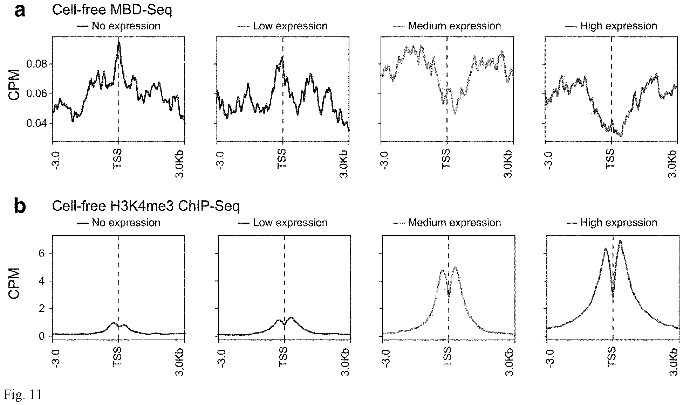

**Fig. 11****: Relationship of cell-free MBD-Seq and cell-free H3K4me3 ChIP-Seq with expression levels of genes.** From publicly available cell-free RNA sequencing data, four groups of genes with different expression levels were defined (no expression (dark blue), low expression (blue), medium expression (red), and high expression (dark red), Figure 3c). (a) Average coverage profiles for cell-free MBD-Seq reads at selected transcription start sites. (b) Average coverage profiles for H3K4me3 ChIP-Seq reads at selected transcription start sites.

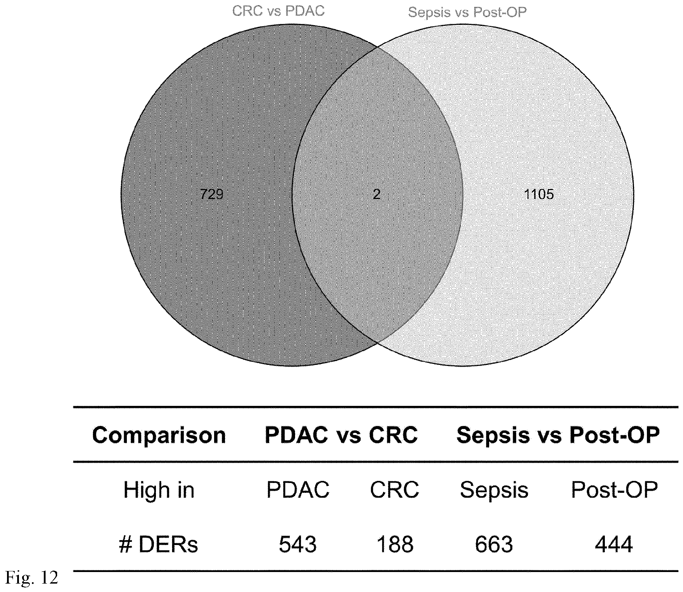

**Fig. 12****:** The table shows the number of differentially enriched regions (DERs) derived from footprint DNA sequencing data for the comparisons of pancreatic ductal adenocarcinoma (PDAC) versus colorectal carcinoma (CRC) and post-operative controls (Post-OP) versus sepsis. The Venn diagram shows the overlap of the two sets of DERs.

### Examples

The following Examples shall merely illustrate the invention. They shall not be construed, whatsoever, to limit the scope of the invention.

### Example 1: Blood samples

This study included blood samples from nine healthy individuals, four individuals with pancreatic ductal adenocarcinoma, four individuals with colorectal carcinoma, four individuals with sepsis, and four individuals that recently underwent surgery in the clinic (Table 1). Blood from healthy individuals was acquired commercially from Biomex GmbH. Septic patients participated in a previously published, prospective observational clinical study which was conducted in the surgical intensive care unit of Heidelberg University Hospital, Germany between November 2013 and January 2015 (German Clinical Trials Register: DRKS00005463). Patients that underwent clinical surgery and all individuals with carcinoma are part of the Immunophenotyping of gastrointestinal cancer disease (ICD) clinical study of the university clinic of Erlangen, which was approved by the Ethics Committee. All experiments were performed in accordance with the study protocol approved by the Ethics Committee.

**Table 1: Metadata for the generated datasets included in this study. The initial sequencing depth for all samples is given in the column 'raw reads ', while the sequencing depth after the samples have been fully processed is given as 'processed reads'.**

| **Sequencing type** | **Sample** | **Condition** | **Raw reads** | **Processed reads** |
|---|---|---|---|---|
| Liquid footprint | S01 | Healthy | 3.22·10⁷ | 5.13·10⁶ |
| Liquid footprint | S02 | Healthy | 1.19·10⁸ | 5.69·10⁶ |
| Liquid footprint | S03 | Healthy | 1.32·10⁸ | 8.29·10⁶ |
| Liquid footprint | S04 | Healthy | 5.11·10⁷ | 5.81·10⁶ |
| Regular cfDNA | S05 | Healthy | 2.65·10⁷ | 2.37·10⁷ |
| Regular cfDNA | S06 | Healthy | 2.95·10⁷ | 2.60·10⁷ |
| Regular cfDNA | S07 | Healthy | 2.55·10⁷ | 2.21·10⁷ |
| Regular cfDNA | S08 | Healthy | 2.54·10⁷ | 2.25·10⁷ |
| Liquid footprint | S09 | Post-OP | 9.52·10⁷ | 1.57·10⁷ |
| Liquid footprint | S10 | Post-OP | 2.13·10⁷ | 5.90·10⁶ |
| Liquid footprint | S11 | Post-OP | 4.80·10⁷ | 1.99·10⁷ |
| Liquid footprint | S12 | Post-OP | 3.82·10⁷ | 1.67·10⁷ |
| Liquid footprint | S13 | Sepsis | 4.67·10⁷ | 1.25·10⁷ |
| Liquid footprint | S14 | Sepsis | 3.20·10⁷ | 1.15·10⁷ |
| Liquid footprint | S15 | Sepsis | 2.54·10⁷ | 1.32·10⁷ |
| Liquid footprint | S16 | Sepsis | 2.92·10⁷ | 1.56·10⁷ |
| Liquid footprint | S17 | PDAC | 3.14·10⁷ | 7.35·10⁶ |
| Liquid footprint | S18 | PDAC | 2.91·10⁷ | 1.08·10⁷ |
| Liquid footprint | S19 | PDAC | 5.41·10⁷ | 2.17·10⁷ |
| Liquid footprint | S20 | PDAC | 4.30·10⁷ | 1.42·10⁷ |
| Liquid footprint | S21 | CRC | 5.85·10⁷ | 2.67·10⁷ |
| Liquid footprint | S22 | CRC | 3.73·10⁷ | 1.82·10⁷ |
| Liquid footprint | S23 | CRC | 4.07·10⁷ | 1.68·10⁷ |
| Liquid footprint | S24 | CRC | 3.97·10⁷ | 2.00·10⁷ |
| MBD-Seq | S25 | Healthy | 7.15·10⁷ | 2.32·10⁷ |

### Example 2: Cell-free DNA isolation

Plasma was prepared by centrifugation of whole blood for 10 min at 1,600 g and 4 °C. Afterward, blood plasma was centrifuged again for 10 min at 16,000 g and 4 °C. Afterward, 1.1 mL of the supernatant was transferred into a fresh 1.5 mL DNA LoBind tube and stored at -80 °C. If necessary, the sample volume was filled up with freshly prepared 1 x phosphate-buffered saline solution. Cell-free DNA was isolated with the QIAsymphony SP DNA Preparation System and the QIAsymphony DSP Circulating DNA Kit (Qiagen) according to the manufacturer's advice. Eluted cfDNA was quantified with the Qubit dsDNA HS Assay Kit (Life Technologies) and the cfDNA quality was assessed by the Fragment Analyzer High Sensitivity DNA Kit (Agilent).

### Example 3: Regular cell-free DNA sequencing

Sequencing libraries for regular cell-free DNA were prepared with the NEBNext Ultra II DNA Library Prep Kit (NEB) according to the manufacturer's protocol. 0.5 ng isolated cfDNA was used as input. The NEBNext Adaptor was diluted 1:25 for all reactions. PCR was performed with 10 PCR cycles and 4 µL primers. Sequencing was performed on a NextSeq 2000 (Illumina) with 100 bp single end reagent kits.

### Example 4: Regular cell-free DNA sequencing data processing

After initial quality control of raw sequencing reads with FastQC (v0.11.8), the following steps were performed38: (1) Removal of sequencing adapters, removal of terminal polyG sequences (min 10 sequential G's), removal of reads shorter than 50 base pairs, and quality trimming (BBTools - bbduk.sh v38.67; Bushnell et al. 2017). (2) Processed reads were mapped to the human reference genome assembly GRCh37 using NextGenMap (v0.5.5) with default settings (Sedlazeck et al., Li et al. 2009, 2013). (3) Mapped reads were deduplicated with samtools rmdup (v1.9), and reads in blacklisted regions were removed with bedtools intersect (v2.30.0; Amemiya et al. 2019, Li et al. 2009, Quinlan & Hall 2010). Mapped reads were converted to BigWig format for visualization and other downstream analyses with deeptools (bin size = 10, normalization = counts per million (CPM), bamCoverage v3.5.1; Ramírez et al. 2016).

### Example 5: Cell-free Methyl-CpG-Binding Domain sequencing and Cell-free Methyl-CpG-Binding Domain sequencing data processing

Methylation enrichment was performed using the EpiMark Methylated DNA Enrichment Kit (NEB) according to the manufacturer's instructions. Two reactions with 5-15 ng of cfDNA input each were performed in parallel per sample. Methylated cfDNA was eluted sequentially from both reactions using the same 50 µL of nuclease-free water to increase yield. Sequencing libraries were prepared with the NEBNext Ultra II DNA Library Prep Kit (NEB) according to the manufacturer's protocol. 50 µL methylated cfDNA from the EpiMark procedure was used as input. The NEBNext Adaptor was diluted 1:25 for all reactions. PCR was performed with 17 PCR cycles and 3 µL primers. Sequencing was performed on a NextSeq 2000 (Illumina) with 100 bp single end reagent kits.

Cell-free Methyl-CpG-Binding Domain sequencing data were processed as regular cell-free DNA sequencing data.

### Example 6: Cell-free RNA sequencing data processing

Fastq files of five publicly available cell-free RNA sequencing datasets of different healthy individuals were obtained from the sequencing read archive (SRA). Accession numbers and unique identifiers are listed in **Table 2.** All samples were sequenced in paired-end end mode with a read length of 150 bp and about 10 million reads per sample on average. After initial quality control of raw sequencing reads with FastQC (v0.11.8), the following steps were performed to obtain read counts for individual genes (Andrews, S. 2010): (1) Removal of sequencing adapters, quality trimming, and removal of reads shorter than 100 base pairs (BBTools - bbduk.sh v38.67; Bushnell et al. (2017). (2) Mapping of processed reads to the human reference genome assembly GRCh37 using NextGenMap (v0.5.5) with default settings, keeping only reads with a MAPQ value greater than 2 (Sedlazeck, et al. 2013). (3) Gene quantification in raw read counts using featureCounts (v2.0.1) with the UCSC Genes annotation (https://hgdownload.soe.ucsc.edu/goldenPath/hg19/bigZips/genes/hg19.knownGene.gtf.gz) (Liao et al. 2014). Only protein-coding genes on the autosomal chromosomes were included in the final readcount matrix to reduce confounding by the gender of sample donors. (4) If required, raw read counts were converted to transcripts per million (TPM) using R.

**Table 2 - Metadata for the utilized public datasets in this study. Cell-free H3K4me3 ChIP-Seq data are available from Zenodo: https://zenodo.org/record/4277001/files/Analysis.tgz?download=1. ATAC-Seq data from ENCODE were downloaded with the GRCh38 assembly and converted locally to the GRCh37 assembly with liftOver.**

| **Data type** | **Sample type** | **Source** | **Project ID** | **File ID** |
|---|---|---|---|---|
| ATAC-Seq | Cell line (GM12878) | ENCODE | ENCSR095QNB | ENCFF646NWY SRR10822588, SRR10822583, SRR10822579, SRR10822594, SRR10822591 |
| Cell-free RNA-Seq | Plasma from healthy individuals | SRA | PRJNA598835 | |
| Cell-free H3K4me3 ChIP-Seq | Plasma from a healthy individual | Zenodo | - | H013.1 |
| DNase-Seq | Cell line (GM12878) | ENCODE | ENCSROOOEMT | ENCFF783ZLL |
| DNase hypersensitive sites | Cell line (GM12878) | ENCODE | ENCSROOOEMT | ENCFF273MW |
| Double-stranded cell-free DNA | Plasma from a healthy individual | SRA | PRJNA291063 | SRR2130050 |
| Single-stranded cell-free DNA | Plasma from a healthy individual | SRA | PRJNA291063 | SRR2130051 |

### Example 7: Footprint DNA sequencing

Sequencing libraries were prepared from 3 ng to 15 ng of cfDNA, depending on the clinical condition, using the NEXTFLEX Cell free DNA-Seq Kit (V2) (NOVA-5150-02) according to the manufacturer's advice with one exception: the final library after PCR amplification was eluted in 20 µL nuclease-free water. Library generation was performed with the Biomek FXP workstation (Beckman Coulter). Library quality was assessed by the Fragment Analyzer High Sensitivity DNA Kit (Agilent) and the concentration was measured by the Qubit dsDNA HS Assay Kit (Life Technologies). Size selection of cfDNA libraries was performed using a BluePippin instrument (Sage Science). To select the footprint DNA portion in the range of 150-200 bp (the range corresponding to short cfDNA fragments ligated to sequencing primers), samples were applied to a 3% agarose BluePippin cassette according to the manufacturer's protocol. Briefly described, samples were filled up with water to 30 µL and were mixed with 10 µL of supplied internal marker (100 bp to 250 bp). 23 µL of the eluted, size-selected sample was reamplified with 25 µL of NEXTFLEX PCR Master Mix 2.0 and 2 µL of 1:10 diluted NEXTFLEX Primer Mix 2.0 as described in the NEXTFLEX Cell free DNA-Seq Kit (V2) (NOVA-5150-02), step C PCR amplification. Afterward, samples were purified with 1.2 × the volume of AMPureXP beads (Beckman Coulter) according to the manufacturer's advice. Size selection performance was evaluated by the Fragment Analyzer High Sensitivity DNA Kit (Agilent). If the sample still contained fragments outside the target range of 150 bp to 200 bp, size selection was repeated as previously described, with the exception that the input sample was adjusted to 30 µL with water and the reamplification step was performed with 5 to 8 cycles. After size selection, sequencing was performed on a NextSeq 2000 (Illumina) with 100 bp single end reagent kits.

### Example 8: Footprint DNA sequencing data processing

After initial quality control of raw sequencing reads with FastQC (v0.11.8), the following steps were performed sequentially to remove sequencing artifacts: (1) Removal of sequencing adapters, terminal polyG sequences (min 10 sequential G's), and quality trimming (BBTools - bbduk.sh v38.67). (2) Removal of terminal single A nucleotide added during library preparation (BBTools - bbduk.sh v38.67). (3) Size selection of sequenced fragments allowing read lengths greater than 20 bp and smaller than 60 bp (BBTools - bbduk.sh v38.67). (4) Removal of sequencing reads with low complexity, i.e. dust scores smaller than 7 (prinseq-lite v0.20.4) (Andrews, S. 2010; Bushnell et al. 2017; Schmieder & Edwards ,2011). (5) Processed reads were mapped to the human reference genome assembly GRCh37 using NextGenMap (v0.5.5) with default settings, Sedlazeck, et al. (2013). (6) Mapped reads were deduplicated with samtools rmdup (v1.9), reads in blacklisted regions were removed with bedtools intersect (v2.30.0), and reads with a MAPQ value lower than 1 were removed with samtools view (v1.9) (Amemiya et al. 2019; Li et al. 2009; Quinlan & Hall, 2010). These reads were converted to BigWig format for visualization and other downstream analyses with deeptools (bin size = 10, normalization = counts per million (CPM), bamCoverage v3.5.1; Ramírez et al. 2016). This workflow is graphically summarized in Figure 7.

### Example 9: Processing of cell-free DNA sequencing data from Snyder et al.

After initial quality control of raw sequencing reads with FastQC (v0.11.8), the following steps were performed: (1) Removal of sequencing adapters and quality trimming (cutadapt v4.0; Martin, M. 2011). (2) Mapping of trimmed reads to the human reference genome assembly GRCh37 using NextGenMap (v0.5.5) with minimum map quality of 10; Sedlazeck, et al. (2013). (3) *In silico* size selection of the mapped reads: 35-80 nt for footprint DNA and 120-180 nt for regular cfDNA. (4) Removal of low complexity reads and de-duplication (prinseq-lite v0.20.4; Schmieder & Edwards, 2011). (5) Removal of reads in blacklisted regions with bedtools intersect (v2.30.0; Quinlan & Hall, 2010). (6) For downstream analysis and visualization BigWig files were generated with deeptools (bin size = 10, normalization = counts per million (CPM), bamCoverage v3.5.1; Ramírez et al. 2016).

### Example 10: Peak calling and nucleosome-free region calling

For footprint DNA sequencing data, narrow peaks and broad peaks were called with MACS2 callpeak (narrow: --nomodel --extsize 32 --call-summits --min-length 30 -q 0.05, broad:-nomodel --extsize 32 --broad --max-gap 100 --min-length 500 --broad-cutoff 0.05)48. Consensus peaks of a condition were identified using R and defined as genomic sites where a narrow peak was identified in at least three of four samples. In addition, consensus peaks less than 31 nucleotides apart were combined. For regular cfDNA, nucleosome-free regions were called on the merged dataset of four biological replicates to increase genome-wide coverage and thus reliability. Nucleosome-free regions were identified with the R packages NucDyn, utilizing nucleR, with default settings; Buitrago et al. (2019). Peaks and nucleosome-free regions were annotated to genomic features in R with the ChIPseeker package and default settings; Yu et al. (2015).

### Example 11: Average coverage profiles and heatmaps

Average coverage profiles and heatmaps were created from BigWig files with deeptools (computeMatrix, plotHeatmap, and plotProfile; v3.5.1; Ramírez et al. 2016). Different genomic reference locations were used for average coverage profiles. For **Figure 2** and **Figure 6****,** validated binding sites of transcription factors were retrieved from Gene Transcription Regulation Database (GTRD; Kolmykov et al. 2021). Here, transcription factor binding sites were ranked according to their robustness across different cell lines and tissues, i.e., the number of tissues and cell lines in which the respective binding site was found. Only the top 1000 binding sites were used for average coverage profiles. Promoter-like structures from ENCODE were converted from GRCh38 to GRCh37 with the liftOver tool from UCSC and used as reference regions in Figure 3a (available at https://www.encodeproject.org/files/ENCFF379UDA/). CpG islands were used as reference regions in Figure 3b (available at UCSC TableBrowser, track name = cpgIslandExt). The heatmaps in Figures 3a and b were clustered in two groups using the implemented k-means clustering based on the line-wise average. Missing data in heatmaps were plotted in black.

### Example 12: Transcription factor motif enrichment analysis

Enriched transcription factor motifs were identified with the AME tool from MEME suite (-scoring avg --method fisher; v5.4.1; McLeay & Bailey, 2010). Input DNA sequences were retrieved from consensus peak sets and analyzed for the enrichment of motifs listed in the HOCOMOCOv11_core_HUMAN_mono database; Kulakovskiy et al. (2018). DNA sequences of consensus peaks from footprint DNA sequencing data of healthy individuals were compared to control sequences, generated by shuffling the letters in the input while preserving the frequencies of k-mers (--shuffle). The proportions of identified transcription factor motif classes and their respective superclasses were summarized in a treemap plot using R and the treemap package (v.2.4.3). Footprint DNA sequencing data from other than healthy states were compared with each other. The DNA sequences underlying each consensus peak set were used as control sequences, e.g. consensus peak DNA sequences from CRC as input compared to consensus peak DNA sequences from PDAC as control.

### Example 13: Differential enrichment analysis

Identification of differentially enriched regions (DERs) was performed with the R package DEBrowser (v1.2.0) using the implemented edgeR method with raw read counts in the combined consensus peak sets of two compared conditions, TMM normalization, a glmLRT, and dispersion = 0 (Kucukural et al. 2019; Robinson et al. 2010). Genes were considered as differentially expressed between two conditions (four biological replicates per condition) with an adjusted p-value smaller than 0.1 and a fold change ≤ -2 or ≥ 2. Volcano plots for the differential enrichment analysis were generated with the R package EnhancedVolcano (v1.8.0). The identified DERs of both comparisons were used for a principal component analysis (PCA), and the first three principal components were visualized in R with pca3d (v0.10.2). Samples of each condition were linked to the centroid of the respective condition with a line.

### Example 14: Results

### Enrichment of cell-free DNA footprint fragments at regulatory genomic regions

Using gel electrophoresis-based size selection to enrich for highly informative short double-stranded cfDNA. Our protocol for enrichment of short double-stranded cfDNA comprises extraction of total cell-free DNA from blood plasma by magnetic bead-based kit followed by double-stranded DNA-specific library preparation (**Figure 5**). To select double-stranded DNA fragments of up to 60 bp in length, two subsequent size selection steps are performed using a preparative gel electrophoresis device (**Figure 6**). After high-throughput sequencing of size-selected libraries, reads were quality-checked to ensure normal size distribution between 20 bp and 60 bp (**Figure 7**). The protocol revealed sequencing reads with a normally distributed mean read length of 37.9 bp (SD = 6.6 bp, n = 2×10⁷ reads uniformly sampled from 20 individuals: four healthy individuals, four patients with pancreatic ductal adenocarcinoma, four patients with colorectal carcinoma, four patients with sepsis, and four post-operative patients; **Figure 8**). Size-selected short double-stranded cfDNA (from now on referred to as `footprint DNA') reads have an elevated average GC content of 57.8 % (SD = 1.9 %, **Figure 9**) in contrast to 40.9 % for average human genomic DNA; Piovesan et al. (2019). To determine the genomic localization and origin of footprint DNA, we mapped footprint DNA to human chromosomes and compared it with sequencing data from regular cfDNA of four other healthy individuals (Table 1). Profiles of footprint DNA and regular cfDNA differed considerably as footprint DNA in contrast to regular cfDNA tends to accumulate either in single narrow peaks or in clusters of narrow peaks (i.e. broad peaks), which are frequently located at transcriptional start sites (TSS) of genes or ChIP-Seq validated transcription factor binding sites (Table 3). Table 3 lists transcription factors whose DNA motif was significantly enriched in footprint DNA consensus peaks from four healthy individuals. Over 200 transcription factor motifs of all nine transcription factor superclasses in the database were identified as enriched in footprint DNA peaks.

**Table 3 Transcription factors whose DNA motif was significantly enriched in footprint DNA consensus peaks from four healthy individuals. Enriched transcription factor motifs were identified with the AME tool from MEME suite with the HOCOMOCOvll core HUMAN mono database. DNA sequences of consensus peaks were analyzed for enrichment in comparison to randomized k-mers of the consensus peaks.**

| **TF**_**Family_Name** | **TF**_**superclass**_**name** | **TF_Family_ Count** |
|---|---|---|
| More than 3 adjacent Zn finger factors | Zn-coordinating DBD | 22 |
| Ets-related factors | Helix-turn-helix domains | 19 |
| 3 Zn finger Krueppel-related factors | Zn-coordinating DBD | 15 |
| Factors with multiple dispersed Zn fingers | Zn-coordinating DBD | 13 |
| bHLH-ZIP factors | Basic domains | 10 |
| Jun-related factors | Basic domains | 9 |
| Tal-related factors | Basic domains | 8 |
| Forkhead box (FOX) factors | Helix-turn-helix domains | 8 |
| Interferon-regulatory factors | Helix-turn-helix domains | 7 |
| STAT factors | Immunoglobulin fold | 7 |
| E2F-related factors | Helix-turn-helix domains | 6 |
| Fos-related factors | Basic domains | 5 |
| Maf-related factors | Basic domains | 5 |
| SOX-related factors | Other all-alpha-helical DBD | 5 |
| CREB-related factors | Basic domains | 4 |
| MyoD / ASC-related factors | Basic domains | 4 |
| PAS domain factors | Basic domains | 4 |
| RFX-related factors | Helix-turn-helix domains | 4 |
| NFAT-related factors | Immunoglobulin fold | 4 |
| E2A-related factors | Basic domains | 3 |
| GATA-type Zn fingers | Zn-coordinating DBD | 3 |
| HOX-related factors | Helix-turn-helix domains | 3 |
| Heteromeric CCAAT-binding factors | Other all-alpha-helical DBD | 3 |
| Regulators of differentiation | alpha-Helices exposed by beta- structures | 3 |
| Runt-related factors | Immunoglobulin fold | 3 |
| RXR-related receptors (NR2) | Zn-coordinating DBD | 2 |
| THAP-related factors | Zn-coordinating DBD | 2 |
| TCF-7-related factors | Other all-alpha-helical DBD | 2 |
| NA | NA | 2 |
| NRF | Yet undefined DBD | 1 |
| B-ATF-related factors | Basic domains | 1 |
| Hairy-related factors | Basic domains | 1 |
| GCNF-related receptors (NR6) | Zn-coordinating DBD | 1 |
| NGFI-B-related receptors (NR4) | Zn-coordinating DBD | 1 |
| Thyroid hormone receptor-related factors (NR1) | Zn-coordinating DBDs | 1 |
| Other factors with up to 3 adjacent Zn fingers | Zn-coordinating DBD | 1 |
| HD-LIM factors | Helix-turn-helix domains | 1 |
| HD-SINE factors | Helix-turn-helix domains | 1 |
| Paired-related HD factors | Helix-turn-helix domains | 1 |
| HSF factors | Helix-turn-helix domains | 1 |
| Myb/SANT domain factors | Helix-turn-helix domains | 1 |
| CSL-related factors | Immunoglobulin fold | 1 |
| NF-kappaB-related factors | Immunoglobulin fold | 1 |
| TBrain-related factors | Immunoglobulin fold | 1 |
| TBX2-related factors | Immunoglobulin fold | 1 |
| SMAD factors | beta-Hairpin exposed by an alpha/beta-scaffold | 1 |
| TBP-related factors | beta-Sheet binding to DNA | 1 |

| | | |
|---|---|---|
| *DBD -DNA-binding domains | | |

To compare broad peaks of footprint DNA with regular cfDNA, nucleosome-free regions (NFRs) should be analyzed, since the absence of regular cfDNA is an indicator of the presence of other DNA-binding proteins. Assignment of broad peaks from footprint DNA and NFRs from regular cfDNA to annotated functional elements of the genome shows that an approximately four- and a six-fold higher proportion of broad peaks are found in promoters (>1 Kb upstream of the TSS) and 5' UTR of genes, respectively. Moreover, the proportion of broad peaks assigned to exons is about four times higher than the proportion of NFRs (Figure 1b). A more detailed examination of the chromosomal coverage for protein-coding genes reveals an opposite occupancy profile between footprint DNA and regular cfDNA. While footprint DNA is strongly enriched at TSS, regular cfDNA is substantially depleted at these sites. In addition, footprint DNA possesses a reciprocal pattern compared to regular cfDNA, with footprint DNA exhibiting an oscillatory pattern that is inverted for 1 Kb downstream of the TSS in regular cfDNA (**Figure 1c**). In addition to enrichment at TSS, footprint DNA also exhibits a prominent signal at DNase-hypersensitive sites (DHS) of a reference annotation from a B-cell line (GM12878), whereas the regular cfDNA signal oscillates at neighboring genomic locations and is depleted at the DHS (Figure 1d). Taken together, footprint DNA accumulates at open chromatin or TSS of genes, whereas regular cfDNA reads, i.e. nucleosomes, are clearly depleted in these regions.

To compare the information from broad peaks of footprint DNA with regular cfDNA, nucleosome-free regions (NFRs) should be analyzed, since the absence of regular cfDNA is an indicator of the presence of other DNA-binding proteins.

### Liquid DNA footprinting detects the binding of various transcription factors

Since footprint DNA could be protected from nuclease digestion by binding to transcription factors, peak regions were examined for their potential to contain transcription factor binding motifs. Accordingly, a consensus peak set was defined from narrow peaks from liquid footprint sequencing data of four healthy individuals. Transcription factor motif enrichment analysis at the genomic locations of these consensus peaks revealed a significant enrichment of 203 transcription factor binding motifs out of 401 in the reference motif database (HOCOMOCO). The identified 203 transcription factor motifs belong to 46 different transcription factor families including nine transcription factor superclasses. Furthermore, ChIP-Seq validated transcription factor binding sites from ENCODE3 reveal clear enrichment signals for Nuclear Factor Erythroid 2 (NFE2), RE1 Silencing Transcription Factor (REST), and Spi-1 Proto-Oncogene (SPI1) in footprint DNA, for example, whereas regular cfDNA is depleted at these binding sites. Overall, the average profile of all ChIP-seq based transcription factor binding sites (TFBS) in ENCODE3 shows a clear enrichment of footprint DNA in contrast to regular cfDNA (**Table** 3). ChIP-Seq validated binding sites of CCCTC-Binding Factor (CTCF) show an even more pronounced signal for footprint DNA (**Figure 2e**). On the contrary, adjacent to these binding sites, regular cfDNA exhibits a regular, high-frequency oscillation signal. In good agreement, DNase-seq data shows a combination of a footprint peak signal with adjacent oscillation patterns, while ATAC-Seq detects a broad peak representative for open chromatin at the CTCF binding sites (**Figure 2e**). Our data suggest that footprint DNA most specifically reveals DNA binding at regulatory sites at high resolution as exemplified for NFE2, REST, or SPI1 **(****Figure 2a****-c, f**). Previously it has been shown that a significant fraction of small cfDNA exists as short single-stranded DNA. To find out what the relationship between single-stranded and double-stranded short cfDNA is we compare our footprint DNA signals at transcription start sites and transcription factor binding sites with the method of Snyder et al. for short cfDNA sequencing (single-stranded library preparation). We clearly found a superior signal-to-noise ratio, allowing the detection of binding signals, e.g. TP53BP1, that are not detectable from short single-stranded DNA of Snyder et al. 2016 (**Figure 10**). Moreover, our data suggest that footprint DNA is not a degradation product of regular cfDNA but rather represents a biological entity of its own, as these fragments occur mainly where regular cfDNA is not present.

### Relationships between footprint DNA signals, markers of epigenetic activation, and transcriptional activity

Given that footprint DNA fragments are most likely derived from the active binding of regulatory proteins and are overrepresented at open chromatin regions, a better understanding of the occurrence of liquid DNA footprints in the context of epigenetic and transcriptional regulation is of basic importance. For determining the relationship between footprint DNA signal strength and promoter activation state, annotated promoter-like structures were assigned to active and inactive promoters based on the cell-free histone 3 lysine 4 triple-methylation (H3K4me3) signal from publically available ChIP-Seq data of a healthy individual11. Potentially active promoters with strong H3K4me3 signal showed a markedly higher occupancy of footprint DNA than promoters with weak or no H3K4me3 signal, whereas regular cfDNA shows exactly the opposite behavior (**Figure 3a**). Moreover, the strongest signals for H3K4me3, representing the nucleosome positions, occur at local minima of footprint DNA and vice versa. DNA methylation is known to be an essential regulator of gene activity and is associated with transcription factor binding and thus potentially DNA footprint signals. Consequently, annotated CpG islands were clustered into methylated and unmethylated CpG islands based on the signal strength of cell-free Methyl-CpG-Binding Domain Sequencing (MBD-Seq) data from a healthy individual. Strongly methylated CpG islands show a weaker accumulation of footprint DNA compared to unmethylated CpG islands, while regular cfDNA again behaves the opposite (**Figure 3b**), clearly showing a strong relationship between DNA methylation and footprint DNA. To further analyze a connection between localized footprint DNA signals and downstream gene transcription we made use of publicly available cell-free RNA-seq data from five healthy individuals Zhu, Y. et al. (2021). Based on the average transcript abundance level of protein-coding genes, four subsets of genes were defined: `no expression', `low expression', `medium expression', and `high expression' genes (Figure 3c). Consistent with the definition of these gene subgroups, H3K4me3 histone modifications increase and DNA methylation levels decrease as expression levels increase at the respective TSS of the genes (Figure 11). Genes with medium or high expression show considerable enrichment of footprint DNA reads at their respective TSS, whereas genes with low or no expression show no substantial enrichment. Regular cfDNA again behaves contrary to footprint DNA and shows a much less pronounced difference between active expression (high and medium) and low - or no expression (Figure 3d). In summary, the signal strength of footprint DNA is higher in active promoters than in inactive promoters, higher in unmethylated CpG islands than in methylated CpG islands, and higher in TSS of actively transcribed genes than in TSS of untranscribed genes. Thus, footprint DNA is enriched at loci with markers for epigenetic activation and transcriptionally active genomic regions.

### Liquid footprinting enables the detection of disease-specific transcription factor binding sites in liquid biopsies

To evaluate the potential of liquid footprinting identification of disease-specific sites, footprint DNA data were generated for four biological replicates of four different clinical indications: two types of gastrointestinal carcinomas (pancreatic ductal adenocarcinoma (PDAC) and colorectal carcinoma (CRC)), as well as sepsis and post-surgery controls (Post-OP, Table 1, Tables 4-7). Post-surgery clinical patients were selected as controls because they show cfDNA concentration levels comparable to septic patients but without septic inflammation. Comparison of consensus peak sets for PDAC vs. CRC, revealed 731 different loci, with at least a two-fold change and an adjusted p-value ≤ 0.1 (**Figure 4a** and **Figure 12**). For 1107 loci, a significant differential enrichment was detected when comparing sepsis with Post-OP (Figure 4b and **Figure 12**). Principal component analysis (PCA) based on all differentially enriched regions (DERs) demonstrated a clear separation of all four clinical indications by the first three principal components (**Figure 4c**). Two exemplary DERs demonstrate a distinct differential DNA footprint near a TSS in the context of a larger locus with transcription factor binding sites (**Figures 4d** **and e**). A differentially occupied transcription factor binding site was detected in the promoter of the lymphocyte cytoprotein 1 (LCP1) gene in CRC patients compared to PDAC patients (**Figure 4d**), whereas another bound transcription factor binding site was detected near the promoter of the Plexin C1 gene (PLXNC1) in sepsis patients in contrast to Post-OP patients (**Figure 4e**). In addition to the differential analysis of signal strength at defined consensus peaks, differential enrichment of transcription factor binding motifs was also analyzed in all consensus peaks. For this purpose, the relative abundance of transcription factor motifs in the consensus peaks of one condition was compared with consensus peaks of the respective condition to be compared. Enrichment of 14 different transcription factor motifs was detected for PDAC in comparison to CRC, 19 for CRC in comparison to PDAC, 14 for sepsis in comparison to Post-OP, and 126 for Post-OP in comparison to sepsis (E-value ≤ 10, **Figures 4f****-i**). The binding motif with the strongest enrichment in PDAC patients compared to CRC patients belongs to the Recombination Signal Binding Protein For Immunoglobulin Kappa J Region (RBPJ; **Figure 4f**), for example. One of the physiological roles of the transcription factor RBPJ is the regulation of early pancreatic cell development. Overall, liquid footprinting enables the detection of condition-specific occupancy of loci or transcription factor binding sites in liquid biopsies, which can be used to discriminate different diseases for diagnostic purposes. Footprint DNA sequencing might also help identify transcription factors that may have physiological relevance to the condition.

**Table 4: Genomic location of the highest DERs identified for PDAC in a comparison of PDAC vs CRC on the human genome. The top 20^{th} DERs are listed with reference to the human genome GenBank accession number = GCA 000001405.1**

| **chromosome number** | **start of DER** | **end of DER** |
|---|---|---|
| chr2 | 1484463 | 1484618 |
| chr1 | 171682320 | 171682367 |
| chr2 | 27874705 | 27874756 |
| chr2 | 228652577 | 228652643 |
| chr11 | 32421733 | 32421874 |
| chr8 | 104261327 | 104261404 |
| chr19 | 56278126 | 56278243 |
| chr7 | 159773 | 159935 |
| chr1 | 90363407 | 90363489 |
| chr12 | 132634212 | 132634310 |
| chr1 | 176737438 | 176737494 |
| chr3 | 50029058 | 50029097 |
| chr10 | 105262915 | 105262974 |
| chr9 | 100979561 | 100979691 |
| chr2 | 238195691 | 238195837 |
| chr11 | 121122655 | 121122736 |
| chr11 | 63388538 | 63388612 |
| chr11 | 123364234 | 123364360 |
| chr12 | 130513851 | 130513921 |
| chr18 | 39745735 | 39745804 |

**Table 5: Genomic location of the highest DERs identified for CRCs in a comparison of PDAC vs CRC on the human genome. The top 20^{th} DERs are listed with reference to the human genome GenBank accession number = GCA 000001405.1**

| **chromosome number** | **start of DER** | **end of DER** |
|---|---|---|
| | | |
| chr10 | 22336459 | 22336496 |
| chr3 | 139108481 | 139108773 |
| chr6 | 20217948 | 20218019 |
| chr1 | 17231210 | 17231694 |
| chr1 | 17084993 | 17085056 |
| chr17 | 21906994 | 21907047 |
| chr3 | 187262132 | 187262197 |
| chr16 | 67936720 | 67936813 |
| chr14 | 59487267 | 59487318 |
| chr2 | 134739936 | 134740019 |
| chr1 | 184083173 | 184083235 |
| chr14 | 88411490 | 88411536 |
| chr8 | 116629766 | 116629833 |
| chr10 | 101595590 | 101595635 |
| chr11 | 134671017 | 134671083 |
| chr2 | 169997079 | 169997160 |
| chr2 | 113735746 | 113735862 |
| chr6 | 88469398 | 88469433 |
| chr13 | 33017366 | 33017460 |
| chr20 | 59773313 | 59773386 |

**Table 6: Genomic location of the highest DERs identified for sepsis in a comparison of sepsis vs POP on the human genome. The top 20^{th} DERs are listed with reference to the human genome GenBank accession number = GCA 000001405.1**

| **chromosome number** | **start of DER** | **end of DER** |
|---|---|---|
| chr21 | 16416777 | 16416848 |
| chr7 | 149757745 | 149757801 |
| chr4 | 154125610 | 154125720 |
| chr15 | 95811185 | 95811302 |
| chr3 | 31547324 | 31547448 |
| chr6 | 38134113 | 38134181 |
| chr9 | 139929825 | 139929896 |
| chr2 | 66265151 | 66265234 |
| chr7 | 28709444 | 28709533 |
| chr12 | 52374226 | 52374357 |
| chr5 | 16707506 | 16707550 |
| chr18 | 77408792 | 77408927 |
| chr1 | 224268453 | 224268498 |
| chr20 | 4594999 | 4595044 |
| chr8 | 141378193 | 141378248 |
| chr10 | 35620129 | 35620189 |
| chr5 | 107188844 | 107188903 |
| chr7 | 30760260 | 30760314 |
| chr6 | 123515848 | 123515901 |
| chr3 | 31988373 | 31988480 |

**Table 7: Genomic location of the highest DERs identified for POP in a comparison of sepsis vs POP on the human genome. The top 20^{th} DERs are listed with reference to the human genome GenBank accession number = GCA 000001405.1**

| **chromosome number** | **start of DER** | **end of DER** |
|---|---|---|
| | | |
| chr12 | 132634232 | 132634311 |
| chr13 | 21951296 | 21951403 |
| chr7 | 65939406 | 65939676 |
| chr1 | 207979805 | 207979868 |
| chr20 | 32237309 | 32237372 |
| chr17 | 76670694 | 76670846 |
| chr5 | 167943625 | 167943661 |
| chr5 | 5140440 | 5140651 |
| chr19 | 15839242 | 15839350 |
| chr2 | 242576101 | 242576197 |
| chr19 | 41107388 | 41107453 |
| chr7 | 98964561 | 98964615 |
| chr5 | 130331330 | 130331438 |
| chr1 | 172002339 | 172002385 |
| chr16 | 71950464 | 71950530 |
| chr14 | 96016408 | 96016495 |
| chr19 | 47734136 | 47734170 |
| chr1 | 68628179 | 68628313 |
| chr1 | 11715054 | 11715171 |
| chr7 | 44349271 | 44349323 |

In this study, we present a novel approach for comprehensive DNA footprinting in liquid biopsies by analysis of short double-stranded plasma cell-free DNA. Our liquid footprinting protocol comprises preparative gel electrophoresis to specifically enrich cfDNA fragments with a mean length of ~40 bp in combination with high throughput next-generation sequencing (**Figures 5, 6, 8**). We observed a strong enrichment of short footprint DNA at the TSS of genes, at which depletion of nucleosomes can be observed for regular cfDNA at the same time (**Figure 1a****-c**). A closer inspection of the average signals at the TSS also showed an oscillation pattern reciprocal to that of regular cfDNA at the 5'UTR. These findings indicate a regular shift of nucleosomes from the promoter to the 5'UTR of genes and binding of regulatory DBPs between displaced nucleosomes as revealed by footprint DNA occupancy. In line with the enrichment of footprint DNA at TSS we also found that footprint DNA fragments showed a higher GC content than the human genome on average (footprint DNA = 57.8 %, human genome = 40.9 %). Gene regulatory elements in humans possess an increased GC content, Benjamini & Speed (2012); Zhang et al. (2004). Consequently, footprint DNA fragments and transcription factor binding sites that are enriched in such regions of the genome, reveal elevated GC contents. In addition to the enrichment at TSS, footprint DNA also accumulates at DNase hypersensitive sites. At these open chromatin locations, regular cfDNA exhibits a superposition signal from high-frequency and low-frequency nucleosomal positioning (Figure 1Fehler! Verweisquelle **konnte nicht gefunden werden.d**), which was previously described by Ulz et al., 2019. In line with that, we observed two different types of footprint DNA signals created by DBPs with a narrow binding signal, such as CTCF (**Figure 2a****-e**), and multiple adjacent protein binding events that result in a broader signal (**Figure 10**). The observed narrow signal of regulatory proteins, like CTCF, SPI1, or REST can be explained by their ability to initiate chromatin remodeling or nucleosome displacement in closed chromatin, which is why they are referred to as pioneer factors; Heinz et al (2010); Barozzi et al. (2014);Fu et al (2008); Vanzan et al. (2021). Pioneer factors can bind directly to closed chromatin without the need or presence of auxiliary proteins. In contrast, the binding sites of regulatory proteins such as MYC or TP53BP1 exhibit a much broader signal. These regulatory proteins appear to bind to loci with open chromatin, accompanied by the presence of additional regulatory proteins (**Figure 10**). Remarkably, we also found a striking correlation between DNA footprint signals and markers representative for transcriptional regulation. In this context, we determined that promoters with H3K4me3 histone modifications which are characteristic for active gene transcription revealed stronger footprint DNA signals than those without (**Figure 3a**). Furthermore, we observed a reciprocal correlation between methylation levels of CpGs and corresponding occupancy by footprint DNA (Figure 3Fehler! Verweisquelle konnte nicht gefunden werden.b). Since unmethylated CpG islands are considered markers of epigenetic activation, depletion of regular cfDNA and the presence of DNA footprinting signals indicate the binding of regulatory activating factors at such loci. Regarding gene expression, footprint DNA signals at the TSS are increased at actively transcribed genes compared to genes with low expression. However, footprint DNA does not strictly capture step-wise dynamics of gene expression levels, rather more it exhibits a binary switch between high and low expression (**Figure 3c****-d**). Overall, footprint DNA is significantly correlated with DNA-methylation signatures, H3K4me3 histone modifications, and transcriptional activity of downstream gene loci. Remarkably, we found consistent enrichment of footprint DNA where regular cfDNA is depleted. We propose that the molecular origin of footprint DNA most likely is cfDNA which has been protected from enzymatic digestion by binding to regulatory proteins, including transcription factors (**Figure 2e** **and f**). In agreement with this model, we found that footprint DNA consensus peaks comprise a plethora of transcription factor motifs and that known transcription factor binding sites revealed clear enrichment for footprint DNA (**Table 3Fehler! Verweisquelle konnte nicht gefunden werden.**). Taken together, our data provide strong evidence that footprint DNA represents a characteristic subset of cfDNA that is distinct from nucleosomal cfDNA and does not originate from the degradation of regular cfDNA, which may enable inference of regulatory DNA binding events on a genome-wide level.

In contrast to footprint DNA, which is characterized by short double-stranded DNA (short dsDNA), publications by Snyder et al (2016), Hudecova et al (2022), and Hisano et al (2021) reported on the presence of short single-stranded DNA (short ssDNA). Contrary to short dsDNA, short ssDNA fragments may represent a significant fraction of total cfDNA comprising as much as 20% of a sequencing library. However, short ssDNA demonstrated that the relative signal strength at transcription start sites or TFBS of proteins, e.g. MYC, is very low compared to short dsDNA **(****Figure 10**). Therefore, we assume that cfDNA originally bound by regulatory DNA-binding proteins is short dsDNA whereas short ssDNA might be of other biological origin or might represent further degradation products with higher signal-to-noise ratios. Hudecova et al., and Hisano et al. suggested that much of the short ssDNA could originate from non-canonical DNA structures, such as G4 quadruplexes, which may largely superimpose signals from regulatory protein DNA-binding events.

Transcription factor motifs from footprint DNA revealed sets of differentially enriched transcription factors between different conditions including samples from septic, colorectal carcinoma (CRC) as well as pancreatic cancer (PDAC) patients, **(****Figure 4f****-i),** indicating the diagnostic potential for liquid biopsy applications. Sepsis is characterized by a complex interplay of inflammatory and anti-inflammatory processes orchestrated by regulators of the immune system. Seven out of the top 10 differentially enriched transcription factors identified in septic samples are linked to regulatory pathways of the immune system. For example, transcription factor 4 (TCF4, also known as E2-2 or ITF-2) regulates genes for the differentiation of dendritic cells into interferon-producing plasmacytoid dendritic cells; Reizis (2010). TCF4 also regulates the immune response as a downstream target of TLR2 signaling to induce the expression of immunoregulatory genes such as interleukin 10 (IL-10; Manoharan et al. 2014). The identified transcription factor ZFX is known to be involved in the maintenance of peripheral T cells as well as expansion and maintenance during B cell development and peripheral homeostasis Smith-Raska et al. (2018). These findings might reveal a possible link between identified transcription factors specific to the regulation of the immune system and sepsis. For CRC patients, specificity protein 1 (SP1) was identified as the top hit, and several Fos/Jun family transcription factors were identified as differentially enriched. SP1 is a ubiquitous transcription factor and mediator of critical physiological pathways including cell cycle, proliferation, and metastasis. SP1 plays a key role in regulating genes involved in colorectal cancer growth and metastasis²⁶. Two members of the Fos/Jun family, FOS Like 1 (FOSL1) and FOS Like 2 (FOSL2) are known to promote tumorigenesis and metastasis in colon cancer; Liu et al. (2021). For PDAC patients, Recombination Signal Binding Protein For Immunoglobulin Kappa J Region (RBPJ) was identified as the corresponding top hit. RBPJ is known to form a heterocomplex with Pancreas Associated Transcription Factor 1a (PTF1A) and their interaction is required in the early stage of pancreatic growth, morphogenesis, and lineage fate decision (Masui et al. 2007). In addition, Peroxisome proliferator-activated receptor gamma (PPARy) and Retinoid X receptor alpha (RXRα) were identified, which are known to form a heterocomplex; Lehrke & Lazar (2005). PPARy is a key regulator of adipocyte differentiation, regulates insulin and adipokine production and secretion, and is associated with PDAC. In addition, RXRα promotes proliferation and inhibits apoptosis of pancreatic cancer cells; Chen et al. (2019). Moreover, we found additional disease-specific differentially occupied loci that may be associated with underlying physiological alterations in the diseases, and enable clear separation of patients using PCA **(****Figure 4a-**e). For example, in colorectal cancer patients, we found differential binding of a DBP near the TSS of the lymphocyte cytosolic protein 1 (*LCP1* also known as *plastin-2*) gene compared with PDAC patients. *LCPI* is associated with colorectal cancer progression, prognosis, and metastasis, and patients with late-stage colorectal cancer have higher expression levels of plastin-2; Ning. et al. (2014). In septic patients, we found a strong signal of footprint DNA near the TSS of the Plexin C1 gene (*PLXNC1*) compared with post-operative controls. PLXNC1 augments adhesion, transmigration, and activation of neutrophils during acute lung injury and is induced during an acute inflammatory response (Granja et al. 2014).

Taken together, analysis of short double-stranded fragments might provide the most accurate picture of a genome-wide transcription factor footprint in liquid biopsies. With the ability to identify disease-specific transcription factor binding site occupation for patient classification, we see great potential in the application of footprint DNA sequencing for liquid biopsy applications.

The results presented in this study are based on comparably small sample numbers. Future validation studies with larger cohort sizes will be useful for clinical validation. In addition to clinical validity and utility, cost-effectiveness is an essential factor for clinical diagnostic methods. Although our workflow already represents a resource-efficient alternative to ultra-deep high-throughput sequencing of total cfDNA, a targeted and even more economical approach might be advantageous for implementation. With the new liquid footprinting approach, that directly maps the binding of regulatory proteins to the genome, we want to add a new tool to liquid biopsy diagnostics that could improve the detection of cancer or enable clinically relevant differential diagnosis of cancer types.

### Literature cited:

Amemiya, et al. (2019) Sci Rep 9, 9354; 10.1038/s41598-019-45839-z
.
Andrews, S. (2010) FASTQC. Babraham Bioinformatics, Babraham Institute, Cambridge, United Kingdom, 2010).
Aucamp et al. (2018) Biol. Rev. 93, 1649-1683.
Barozzi et al. (2014) Molecular cell 54, 844-857.
Benjamini & Speed (2012) Nucleic Acids Research 40, e72.
Buitrago et al. (2019) Nucleic Acids Research 47, 9511-9523.
Bushnell et al. (2017) PloS one 12, e0185056.
Burnham, et al. (2016) Scientific Reports 6, 27859; 10.1038/srep27859.
Chen et al. (2019) European review for medical and pharmacological sciences 23.
Fu et al (2008) PLoS genetics 4, e1000138.
Granja et al. (2014) Mucosal immunology 7, 879-891.
Heinz et al (2010) Molecular cell 38, 576-589.
Heitzer et al. (2020) Trends in molecular medicine 26, 519-528.
Hisano et al. (2021) BMC biology 19, 225.
Hudecova et al. (2022) Genome research 32, 215-227.
Kolmykov et al. (2021) Nucleic Acids Research 49, D104-D 111.
Kucukural et al. (2019) BMC genomics 20, 6.
Kulakovskiy et al. (2018) Nucleic Acids Research 46, D252-D259.
Lehrke & Lazar (2005) Cell 123, 993-999.
Li et al. (2009) Bioinformatics (Oxford, England) 25, 2078-2079.
Liu et al. (2021) Pharmacological research 165, 105405.
Liao et al. (2014) Bioinformatics (Oxford, England) 30, 923-930.
Lo et al. (2021) Science 372; 10.1126/science.aaw3616.
Manoharan et al. (2014) Journal of immunology 193, 4203-4213.
Martin, M. (2011) EMBnet j. 17, 10.
Masui et al. (2007) Genes & development 21, 2629-2643.
McLeay & Bailey (2010) BMC bioinformatics 11, 165.
Ning. et al. (2014) Molecular cancer therapeutics 13, 528-539.
Piovesan et al. (2019) BMC research notes 12, 106.
Ramirez et al. (2016) Nucleic Acids Research 44, W160-5.
Reizis (2010) Current opinion in immunology 22, 206-211.
Robinson et al. (2010) Bioinformatics (Oxford, England) 26, 139-140.
Schmieder & Edwards (2011) Bioinformatics (Oxford, England) 27, 863-864.
Sedlazeck, et al. (2013) Bioinformatics (Oxford, England) 29, 2790-2791.
Smith-Raska et al. (2018) Frontiers in immunology 9, 1482.
Snyder et al. (2016) Cell 164, 57-68; 10.
Sun, et al. (2015) PNAS 112, E5503-12.
Quinlan & Hall (2010) Bioinformatics (Oxford, England) 26, 841-842.
Ulz et al. (2019) Nat Commun 10, 4666; 10.1038/s41467-019-12714-4.
Vanzan et al. (2021) Nat Commun 12, 3337.
Yu et al. (2015) Bioinformatics (Oxford, England) 31, 2382-2383.
Zhang et al. (2004) PNAS 101, 16855-16860.
Zhu, Y. et al. (2021) Theranostics 11, 181-193.

## Claims

1. A method for identifying a cfDNA derived biomarker for a physiological condition, disease or disorder of interest, comprising:
a) isolating cfDNA from a sample containing cfDNA of a subject known to be in the physiological condition of interest or to suffer from the disease or disorder of interest;
b) selecting short double-stranded cfDNA fragments;
c) determining the nucleic acid sequences of said short double-stranded cfDNA fragments;
d) comparing the nucleic acid sequences of said short double-stranded cfDNA fragments to a reference; and;
e) based on said comparison in step d) identifying at least one cfDNA derived biomarker associated with the physiological condition, disease or disorder of interest.

2. The method as claimed in claim 1, wherein the short double-stranded cfDNA fragments are double-stranded DNA fragments with a fragment size below 100 bp, preferably between 10 and 80 bp, more preferably between 20 and 60 bp.

3. The method as claimed in claim 1 or 2, wherein the sample is a sample of a body fluid, preferably selected form the group consisting of blood, plasma, serum, lacrimal fluid, urine, lymph, cerebrospinal fluid, bile, stool, sweat, amniotic fluid, synovial fluid and saliva, more preferably the sample is a plasma sample.

4. The method as claimed in any of claims 1 to 3, wherein said selecting short double-stranded cfDNA fragments comprises gel electrophoretic size separation of the double-stranded cfDNA, gradient gel electrophoresis, PCR-based approaches, hybridization and capture, and/or selection using binding protein crosslinking and capture, preferably gel electrophoretic size separation of a sequencing library of the double-stranded cfDNA.

5. The method as claimed in any of claims 1 to 4, wherein the determined nucleic acid sequences comprise protein binding footprint DNA sequences including protein-DNA interaction sites such as transcription factor (TF) binding sites, binding sites of structural proteins, binding site of regulatory proteins, transcriptional start sites, or gene promoter regions, CpG islands, preferably TF binding sites.

6. The method as claimed in any of claims 1 to 5, wherein the reference comprises one or more short double-stranded cfDNA fragments that are (i) derived from a sample of a body fluid from a healthy subject or a sample of a body fluid from a subject not being in the physiological condition of interest or not suffering from the disease or disorder of interest, or (ii) are known short double-stranded cfDNA fragments.

7. The method as claimed in any of claims 1 to 6, wherein the physiological condition, disease or disorder of interest is selected from the group consisting of:
i) cancer including pancreatic ductal adenocarcinoma (PDAC), colorectal carcinoma (CRC), non-small cell lung cancer (NSCLC), bone cancer;
ii) fungal, parasitic, viral or bacterial infections including sepsis, pancreatitis, lyme disease, encephalitis;
iii) chronic inflammatory diseases including chronic inflammatory bowel disease (IBD) such as ulcerative colitis or Crohn's disease;
iv) autoimmune diseases including multiple sclerosis (MS), rheumatism;
v) metabolic disorder;
vi) irritable bowel syndrome;
vii) genetic diseases or developmental aberrations in particular of the fetus including trisomies; and
viii) pregnancy, developmental stages;
preferably wherein the disease or disorder is a cancer, or an infectious disease, more preferably selected from pancreatic ductal adenocarinoma (PDAC), colorectal carcinoma (CRC), and sepsis.

8. The method as claimed in any of claims 1 to 7, further comprising a step of mapping the determined nucleic acid sequences of the short double-stranded cfDNA fragments to a reference genome, preferably thereby obtaining mapped sequences.

9. The method as claimed in any of claims 1-8, comprising identifying differentially enriched regions (DERs), typically by said comparison of the nucleic acid sequences of said short double-stranded cfDNA fragments of a sample to a reference.

10. The method as claimed in claim 8, wherein the at least one disease or disorder associated cfDNA derived biomarker is based on a combination of more than one of the DERs.

11. A method for assessing a physiological condition, disease or disorder of interest in a sample of a subject suspected to be in said condition or to suffer from said disease or disorder of interest, comprising:
a) isolating cfDNA from a sample containing cfDNA of said subject;
b) selecting short double-stranded cfDNA fragments;
c) determining the nucleic acid sequences of said short double-stranded cfDNA fragments; and
d) determining the presence, absence or abundance of at least one cfDNA derived biomarker associated with the physiological condition, disease or disorder of interest within the determined of the nucleic acid sequences of said short double-stranded cfDNA fragments;
preferably wherein the at least one cfDNA derived biomarker associated with the physiological condition, disease or disorder of interest is identified or is identifiable by the method as claimed in any one of claims 1 to 10.

12. A method for determining efficacy of treating a physiological condition, disease or disorder of interest in a sample of a subject known to be in said condition or suffer from said disease or disorder comprising:
a) isolating cfDNA from a sample containing cfDNA of said subject;
b) selecting short double-stranded cfDNA fragments;
c) determining the nucleic acid sequences of said short double-stranded cfDNA fragments;
d) determining the presence, absence or abundance of at least one cfDNA derived biomarker associated with the physiological condition disease, or disorder of interest within the determined nucleic acid sequences of said short double-stranded cfDNA fragments;
e) repeating steps a) to d); and
f) determining efficacy of therapy based on the determination over time.

13. A device for detecting at least one cfDNA derived biomarker in a liquid biopsy sample comprising at least:
1) a sample collector and selector for isolating and selecting cfDNA;
2) a sequencer, preferably, for high-throughput sequence; and
3) an evaluating unit comprising at least a processor.

14. Use of a cfDNA derived biomarker, preferably obtained by the method of any one of claims 1 to 10, in a sample for assessing a disease or disorder in a subject suspected to suffer therefrom.

15. A cfDNA derived biomarker, preferably obtained by the method of any one of claims 1 to 10, for use in diagnosing a disease or disorder in a subject suspected to suffer therefrom.
